(19) Europäisches Patentamt / European Patent Office / Office européen des brevets

(11) **EP 4 105 237 A1**

(12) **EUROPEAN PATENT APPLICATION**
published in accordance with Art. 153(4) EPC

(43) Date of publication:
**21.12.2022 Bulletin 2022/51**

(21) Application number: **21753717.4**

(22) Date of filing: **10.02.2021**

(51) International Patent Classification (IPC):
*C07K 16/30* $^{(2006.01)}$     *A61K 39/395* $^{(2006.01)}$
*A61K 39/00* $^{(2006.01)}$     *A61P 35/00* $^{(2006.01)}$

(52) Cooperative Patent Classification (CPC):
**A61K 39/00; A61K 39/395; A61P 35/00;
C07K 16/30**

(86) International application number:
**PCT/CN2021/076481**

(87) International publication number:
**WO 2021/160154 (19.08.2021 Gazette 2021/33)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.02.2020 CN 202010084476**

(71) Applicant: **Shanghai Escugen Biotechnology Co.,
Ltd.
Shanghai 201203 (CN)**

(72) Inventors:
• **ZHOU, Qing
Shanghai 201203 (CN)**
• **XU, Chuanying
Shanghai 201203 (CN)**
• **NIAN, Weihong
Shanghai 201203 (CN)**

• **HE, Xiangyu
Shanghai 201203 (CN)**
• **ZHANG, Xinmin
Shanghai 201203 (CN)**
• **ZHENG, Xintong
Shanghai 201203 (CN)**
• **HE, Feng
Shanghai 201203 (CN)**
• **XIAO, Jing
Shanghai 201203 (CN)**

(74) Representative: **Abel & Imray LLP
Westpoint Building
James Street West
Bath BA1 2DA (GB)**

Remarks:
The complete document including Reference
Table(s) and the Sequence Listing(s) can be
downloaded from the EPO website

(54) **CLDN18.2 ANTIBODY AND USE THEREOF**

(57)     Provided are an antibody against claudin 18.2 (cldn18.2) and a use thereof in diagnosing and treating cancer of the stomach, pancreas and esophagus.

**Description**

**TECHNICAL FIELD OF THE APPLICATION**

The present application relates to the technical field of antibodies, as well as the use and preparation method of antibodies. Specifically, it relates to a CLDN18.2 antibody and use thereof.

**BACKGROUND OF THE APPLICATION**

Claudin is a class of transmembrane proteins with a molecular weight of 20 to 30 kDa. Its family has 24 members which are expressed in many tissues and are important molecules for the formation of tight junctions (TJ) between cells. Epithelial cells or endothelial cells form a highly ordered organization of binding through tight-binding molecules (including Claudin molecules) expressed on the cell surface, that is, tight junctions between cells, which control the flow of molecules in the epithelium space. TJ plays a crucial role in maintaining the stable state of epithelial cells or endothelial cells under normal physiological conditions and maintaining cell polarity. Among them, more striking is claudin 18 (CLDN18), which includes two variants formed by splicing: CLDN18 splice variant 1 (Claudin18.1, CLDN18.1) and CLDN18 splice variant 2 (Claudin 18.2, CLDN 18.2).

Among them, CLDN18.2 (Genbank accession numbers NM_001002026, and NP_001002026) is a transmembrane protein with a molecular weight of 27.8 kDa, and both the N-terminal and C-terminal of the protein are located intracellularly. The main conformation of the protein comprises 4 transmembrane domains (TMD) and 2 extracellular loops (ECL). CLDN18.2 is highly conserved in a variety of mammals, with a full length of 261 amino acids, of which amino acids 1-6 are the N-terminal intracellular domain, amino acids 7-27 are the transmembrane domain 1 (TMD1), amino acids 28-78 are the extracellular loop 1 (ECL1), amino acids 79-99 are the transmembrane domain 2 (TMD2), amino acids 123-144 are the transmembrane domain 3 (TMD3), amino acids 100-122 are the intracellular region connecting TMD2 and TMD3, amino acids 145-167 are the extracellular loop 2 (ECL2), amino acids 168-190 are the transmembrane region 4 (TMD4), and amino acids 191-261 are the C-terminal intracellular region. There is a classical N-glycosylation motif on the asparagine at position 116.

CLDN18.1 and CLDN18.2 differ only in the first 26 amino acids of the N-terminal in the N-terminal-transmembrane region 1-extracellular loop 1 of the protein, and the rest of the sequences are the same.

There is considerable evidence suggesting that CLDN18.2 is highly expressed in tumor cells of stomach, pancreas, esophagus and other tissues, especially in adenocarcinoma subtype tumor cells. In normal tissues, CLDN18.1 is selectively expressed in lung and gastric epithelial cells; CLDN18.2 is only expressed in short-lived gastric glandular epithelial mucosal tissue (differentiated glandular epithelial cells), but not in gastric epithelial stem cells, and differentiated glandular epithelial cells can be continuously replenished by gastric epithelial stem cells. These molecular expression characteristics provide potential for antibody-based treatment of CLDN18.2-related cancers.

The use of companion diagnostics (CDx) methods to select tumor patients with high expression of CLDN18.2 in clinical trials and clinical applications has important guiding significance for guiding the applicable patients of anti-tumor drugs targeting CLDN18.2. In clinical diagnosis of tumor-specific antigens, patient tumor specimens are usually formalin fixed paraffin-embedded (FFPE) tissues, and tumor-specific antigens will change their natural conformation during FFPE processing. In the subsequent antigen retrieval process, special epitopes that are different from the natural conformation will be exposed (Biotech Histochem, 2009, 84(5): 207-215), therefore diagnosis is typically performed by screening for antibodies that bind specifically to cancer-specific antigens on tumor FFPE tissue.

The application relates to the preparation method, screening, sequence and use of an antibody that specifically recognizes CLDN18.2 antigen in tumor and its FFPE tissue.

**SUMMARY OF THE APPLICATION**

The present application provides a CLDN18.2 antibody for diagnosing cancer, the antibody can recognize CLDN18.2 antigen molecules in fixed tissue samples to diagnose cancer lesions of that including gastric cancer, pancreatic cancer or esophageal cancer. Meanwhile, the CLDN18.2 antibody provided in the present application can also effectively treat cancer lesions of that including gastric cancer, pancreatic cancer or esophageal cancer. Specifically, the application relates to:

1. A CLDN18.2 antibody comprising heavy chain CDRs and light chain CDRs, wherein the heavy chain CDRs are:

CDR1 comprising the sequence as shown in SEQ ID NO: 18, CDR2 comprising the sequence as shown in SEQ ID NO: 19, and CDR3 comprising the sequence as shown in SEQ ID NO:20; or

CDR1 comprising the sequence as shown in SEQ ID NO:21, CDR2 comprising the sequence as shown in SEQ ID NO:22, and CDR3 comprising the sequence as shown in SEQ ID NO:23,
wherein the light chain CDRs are:
CDR1 comprising the sequence as shown in SEQ ID NO:24, CDR2 comprising the sequence as shown in SEQ ID NO:25, and CDR3 comprising the sequence as shown in SEQ ID NO:26; or CDR1 comprising the sequence as shown in SEQ ID NO:27, CDR2 comprising the sequence as shown in SEQ ID NO:28, and CDR3 comprising the sequence as shown in SEQ ID NO: 29.

2. The antibody of item 1, comprising a heavy chain variable region as shown in SEQ ID NO:36 or SEQ ID NO:37.

3. The antibody of item 1, comprising a light chain variable region as shown in SEQ ID NO:38 or SEQ ID NO:39.

4. The antibody of any one of items 1-3, comprising a heavy chain variable region as shown in SEQ ID NO:36 and a light chain variable region as shown in SEQ ID NO:38.

5. The antibody of any one of items 1-3, comprising a heavy chain variable region as shown in SEQ ID NO:37 and a light chain variable region as shown in SEQ ID NO:39.

6. The antibody of any one of items 1-5, which is a chimeric antibody, a humanized antibody, or a fully human antibody.

7. The antibody of any one of items 1-6, which is a full-length antibody.

8. The antibody of item 7, which is an IgG antibody.

9. The antibody of any one of items 1-6, which is an antibody fragment that binds to CLDN18.2.

10. The antibody of item 9, which is Fab, Fab'-SH, Fv, scFv or $(Fab')_2$.

11. The antibody of any one of items 1-8, which is a multispecific antibody or a bispecific antibody.

12. The antibody of item 11, wherein the multispecific or bispecific antibody comprises a binding domain that binds to a second biomolecule, wherein the second biomolecule is a cell surface antigen.

13. The antibody of item 12, wherein the cell surface antigen is a tumor antigen.

14. The antibody of item 13, wherein the tumor antigen is selected from the group consisting of: CD3, CD20, FcRH5, HER2, LYPD1, LY6G6D, PMEL17, LY6E, CD19, CD33, CD22, CD79A, CD79B, EDAR, GFRA1, MRP4, RET, Steap1 and TenB2.

15. An immunoconjugate, comprising a therapeutic agent, an agonist of stimulator of interferon genes (STING) receptor, a cytokine, a radionuclide or an enzyme linked to the antibody of any one of items 1-10.

16. The immunoconjugate of item 15, wherein the therapeutic agent is a chemotherapeutic drug.

17. The immunoconjugate of item 15, wherein the therapeutic agent is a cytotoxic agent.

18. A fusion protein or polypeptide comprising the antibody of any one of items 1-10 or an antigen-binding fragment thereof.

19. A pharmaceutical composition comprising the antibody of any one of items 1-14, the immunoconjugate of any one of items 15-17, or the fusion protein or polypeptide of item 18.

20. An isolated nucleic acid comprising a polynucleotide sequence encoding the amino acid sequence of any one of SEQ ID NOs: 18-29.

21. The nucleic acid of item 20, comprising a polynucleotide sequence encoding the amino acid sequence of any one of SEQ ID NOs: 36-39.

22. The nucleic acid of item 21, comprising a polynucleotide sequence encoding the antibody of any one of items 1-10 or an antigen-binding fragment thereof.

23. A vector comprising the polynucleotide sequence of any one of items 20-22.

24. A host cell comprising the polynucleotide sequence of any one of items 20-22 or the vector of item 23.

25. A method for preparing the antibody of any one of items 1-10, comprising culturing the host cell of item 24 and isolating the antibody from the culture.

26. Use of a pharmaceutical composition comprising the antibody of any one of items 1-14, the immunoconjugate of any one of items 15-17, or the fusion protein or polypeptide of item 18 in the preparation of a reagent for diagnosing cancer.

27. Use of a pharmaceutical composition comprising the antibody of any one of items 1-14, the immunoconjugate of any one of items 15-17, or the fusion protein or polypeptide of item 18 in the preparation of a medicament for treating cancer.

28. The use of item 26 or 27, wherein the cancer is gastric cancer, pancreatic cancer or esophageal cancer.

29. A cancer detection reagent comprising the antibody of any one of items 1-10 or an antigen-binding fragment thereof.

30. The cancer detection reagent of item 29, wherein the antibody or antigen-binding fragment thereof is chemically labeled.

31. The cancer detection reagent of item 30, wherein the label is an enzymatic label, a fluorescent label, an isotopic label or a chemiluminescent label.

32. A cancer diagnosis kit comprising the cancer detection reagent of any one of items 29-31.

33. The kit of item 32, wherein the cancer is gastric cancer, pancreatic cancer or esophageal cancer.

34. A method for diagnosing cancer in a subject, comprising contacting the antibody of any one of items 1-14, the fusion protein or polypeptide of item 18, or the detection reagent of any one of items 29-31 with a tissue sample derived from the subject.

35. The diagnostic method of item 34, wherein the tissue sample is body fluid (e.g., blood, and urine) and a tissue section (e.g., tissue biopsy sample) of the subject.

36. The diagnostic method of item 34 or 35, wherein the cancer is selected from gastric cancer, pancreatic cancer or esophageal cancer.

37. A method for treating cancer in a subject, comprising administering to the subject a therapeutically effective amount of the antibody of any one of items 1-14, the immunoconjugate of any one of items 15-17, the fusion protein or polypeptide of item 18, or the pharmaceutical composition of item 19.

38. The treatment method of item 37, wherein the cancer is selected from gastric cancer, pancreatic cancer or esophageal cancer.

**BRIEF DESCRIPTION OF THE DRAWINGS**

Figure 1. Transient transfection of HEK293-T cells with human hCLDN18.2-TCE (T-cell epitope peptide) retrovirus expression plasmid.

Figure 2. Transfection of mouse tumor cell lines with hCLDN18.2-TCE lentiviral particles and sorting of mouse tumor cells expressing high levels of hCLDN18.2-TCE by flow cytometry.

Figure 3. Flow cytometric analysis of hCLDN18.2, hCLDN18.1, mCLDN18.2, and mCLDN18.1 stably transfected cell lines.

Figure 4. SDS-PAGE detection of the positive control antibodies 43A11, 175D10 and 163E12.

Figure 5. Binding of the supernatant of positive hybridoma clones to HEK293-hCLDN18.2 cells and HEK293-hCLDN18.1 cells in confirmation screening.

Figure 6. Flow cytometry analysis of the supernatant of some positive hybridoma clones on 5 cell lines in validation screening.

Figure 7. ADCC effect of the supernatant of some positive hybridoma clones.

Figure 8. IHC staining of the positive control antibody on paraffin-embedded (FFPE) tissue sections of the HEK293-hCLDN18.2 (A), HEK293-hCLDN18.1 (B) and HEK293-WT (C) cell blocks.

Figure 9. IHC staining of the supernatant of 5-15 hybridoma clone and the positive control antibody on paraffin-embedded (FFPE) tissue sections of the gastric cancer tissue.

Figure 10. Flow cytometry analysis of the hybridoma monoclonal supernatant on HEK293-hCLDN18.2 cells.

Figure 11. Reduced SDS-PAGE electrophoresis of the purified recombinant mouse monoclonal antibodies 5-110 and 5-G17.

Figure 12. Flow cytometric analysis of the purified recombinant monoclonal antibodies 5-110 and 5-G17 in HEK293-hCLDN18.2.

Figure 13. Positive staining of the recombinant purified mouse monoclonal antibodies 5-110 and 5-G17 on gastric cancer FFPE tissue sections.

**DETAIL DESCRIPTION OF THE APPLICATION**

1. Definition

The term "antibody" is used herein in a broad sense to encompass various antibody structural molecules that bind CLDN18.2 and comprise one or more of the CDR domains disclosed herein, including but not limited to monoclonal antibodies, polyclonal antibodies, multispecific antibodies (e.g., bispecific antibodies) as well as antibody fragments (e.g., Fv, Fab, Fab', Fab'-SH, and F(ab')$_2$), linear antibodies and single chain antibody molecules (e.g., scFv) etc. as long as it exhibits the desired binding activity to CLDN18.2.

Those skilled in the art can fuse one or more CDR domains disclosed in the present application with one or more other polypeptide sequences to prepare functional fusion proteins or polypeptide molecules that bind to CLDN18.2 molecules, such as vaccines, cell membrane receptor antagonists, signaling pathway modulators and chimeric antigen receptor molecules, etc. For example, one or more CDR domains disclosed in the present application can be used to prepare a CLDN18.2 CAR-T (Chimeric Antigen Receptor T-Cell Immunotherapy) molecule. These fusion protein molecules derived and prepared based on the sequence disclosed in the present application are also included in the protection scope of the present application.

The modifier "monoclonal" in the term "monoclonal antibody" as used herein means that the antibody is obtained from a substantially homogeneous population of antibodies, comprising only trace amounts of naturally occurring mutations or those that occur during the preparation of monoclonal antibodies. Compared to polyclonal antibody preparations that typically include different antibodies specific to different epitopes, each monoclonal antibody in a monoclonal antibody preparation is specific to a single epitope on an antigen. Monoclonal antibodies of the application can be made by a variety of techniques including, but not limited to, hybridoma methods, recombinant DNA methods, phage display methods, and methods using transgenic animals comprising all or part of the human immunoglobulin loci.

The terms "full-length antibody" and "intact antibody" refer to an antibody having a structure substantially similar to that of a natural antibody, and the terms are used interchangeably herein.

The "class" of an antibody refers to the type of the constant domain or constant region possessed by its heavy chain. There are five major classes of antibodies: IgA, IgD, IgE, IgG, and IgM, and several of these can be further divided into subclasses (isotypes), e.g., IgG1, IgG2, IgG3, IgG4, IgA1, and IgA2. The heavy chain constant domains corresponding to the different classes of immunoglobulins are called $\alpha$, $\delta$, $\varepsilon$, $\gamma$ and $\mu$, respectively.

A "chimeric antibody" is an antibody having at least a portion of a heavy chain variable region and at least a portion of a light chain variable region derived from one species and at least a portion of a constant region derived from another species. For example, in one embodiment, the chimeric antibody may comprise murine variable regions and human constant regions.

A "humanized" antibody refers to a chimeric antibody comprising amino acid residues from a non-human HVR and amino acid residues from a human FR. In certain embodiments, the humanized antibody comprises at least one, usually two, or substantially an entire variable domain, wherein all or substantially all HVRs (e.g., CDRs) correspond to those of the non-human antibody, and all or substantially all FRs correspond to those of the human antibody. Optionally, the humanized antibody may comprise at least a portion of an antibody constant region derived from the human antibody. A "humanized form" of the antibody (e.g., a non-human antibody) refers to an antibody that has undergone humanization.

A "human common framework" is a framework that represents the amino acid residues most frequently found in the selection of human immunoglobulin VL or VH framework sequences. Generally, human immunoglobulin VL or VH sequences are selected from a subgroup of variable domain sequences. Generally, the subgroup of sequences is as in Kabat et al., Sequences of Proteins of Immunological Interest, 5th edition, NIH Publication 91-3242, Bethesda MD (1991), vols. 1-3. In one embodiment, for VL, the subgroup is subgroup $\kappa$I as described by Kabat et al. (supra). In one embodiment, for VH, the subgroup is subgroup III as described by Kabat et al. (supra).

A "human being antibody" which may also be referred to as a "human antibody," "fully human source antibody," or "fully human antibody" is an antibody whose amino acid sequence corresponds to that produced by humans or by human cells. This definition of human antibody specifically excludes humanized antibodies comprising non-human antigen-binding residues. Human antibodies can be prepared using a variety of techniques known in the art, including phage display library techniques, as described in Hoogenboom and Winter, J. Mol. Biol., 227: 381 (1991); Marks et al., J. Mol. Biol., 222: 581 (1991); Cole et al., Monoclonal Antibodies and Cancer Therapy, Alan R. Liss, p. 77 (1985); Boerner et al., J. Immunol., 147(1): 86-95 (1991). Human antibodies can be prepared by administering antigen to transgenic animals (e.g., immunized xenogeneic mice) that have been modified to produce such antibodies in response to antigenic challenge, but whose endogenous loci have been disabled (for XENOMOUSE™ technology, see, e.g., US Pat. Nos. 6,075,181 and 6,150,584). For human antibodies produced via human B cell hybridoma technology, see also, e.g., Li et al., Proc. Natl. Acad. Sci. USA, 103: 3557-3562 (2006).

The term "hypervariable region" or "HVR" as used herein refers to a region of the variable domain of an antibody having a sequence hypervariable region (also referred to as "complementarity determining region" or "CDR") and/or forming a structurally defined loop ("hypervariable loop") and/or comprising an antigen-contacting residue ("antigen contact"). In general, the antibody comprises 6 HVRs (CDR regions): 3 in the VH (HI, H2, and H3) and 3 in the VL (L1, L2, and L3). Exemplary HVRs (CDR regions) herein include:

(a) hypervariable loops occurring at amino acid residues 26-32 (L1), 50-52 (L2), 91-96 (L3), 26-32 (HI), 53-55 (H2), and 96-101 (H3) (Chothia and Lesk, J. Mol. Biol. 196: 901-917 (1987));
(b) CDRs occurring at amino acid residues 24-34 (L1), 50-56 (L2), 89-97 (L3), 31-35b (HI), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (HI), 47-58 (H2), and 93-101 (H3) (MacCallum et al. J. Mol. Biol. 262: 732-745 (1996)); and
(d) combinations of (a), (b), and/or (c), including HVR (CDR region) amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR (CDR region) residues and other residues in the variable domain (e.g., FR residues) are numbered herein according to Kabat et al. (supra).

The term "variable region" or "variable domain" refers to the antibody heavy or light chain domain involved in binding an antibody to an antigen. The variable domains of the heavy and light chains (VH and VL, respectively) of natural antibodies generally have similar structures, with each domain comprising 4 conserved framework regions (FRs) and 3 complementarity determining regions (CDR regions). (See, e.g., Kindt et al., Kuby Immunology, 6th ed.) A single VH or VL domain may be sufficient to confer antigen-binding specificity.

An "antibody fragment" refers to a molecule other than an intact antibody that comprises a portion of an intact antibody that binds the antigen to which the intact antibody binds. Examples of antibody fragments include, but are not limited to, Fv, Fab, Fab', Fab'-SH, $F(ab')_2$; diabodies; linear antibodies; single-chain antibody molecules (e.g., scFv); and multispecific antibodies formed from antibody fragments.

The term "cytotoxic agent" as used herein refers to a substance that inhibits or prevents cell function and/or causes cell death or destruction. Cytotoxic agents include, but are not limited to, radioisotopes (e.g., radioisotopes of At211, 1131, 1125, Y90, Re186, Re188, Sm153, Bi212, P32, Pb212, and Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof, such as nucleolytic enzymes; antibiotics; toxins, such as small molecule toxins or enzymatically active toxins of bacterial, fungal, plant or animal origin, including fragments and/or variants thereof; various antitumor drugs or anticancer agents known in the art.

An "immunoconjugate" is a conjugate of an antibody and one or more heterologous molecules, including but not limited to cytotoxic agents.

A stimulator of interferon genes (STING) receptor agonist is a molecule that activates the STING-dependent signaling pathway to promote the secretion of type I interferon and the expression of proteins related to anti-viral and anti-tumor immunity, block virus replication, and promote the immune response to cancer cells. Such molecules are STING agonists of structural classes such as cyclic dinucleotides, aminobenzimidazoles, xanthones, acridinones, benzothiophenes and benzodioxoles.

A "subject" or "individual" is a mammal. Mammals include, but are not limited to, domesticated animals (e.g., cows, sheep, cats, dogs, and horses), primates (e.g., humans and non-human primates, such as monkeys), rabbits, and rodents (e.g., mice and rats). In certain embodiments, the subject or individual is a human.

The term "package insert" is used to refer to instructions typically included in commercial packages of therapeutic products, which comprise information on indications, usage, dosage, administration, combination therapies, contraindications and/or warnings regarding the use of such therapeutic products.

"Affinity" refers to the strength of the sum of non-covalent interactions between a single binding site of a molecule (e.g., an antibody) and its binding partner (e.g., an antigen). Unless otherwise indicated, as used herein, "binding affinity" refers to intrinsic binding affinity, which reflects a 1:1 interaction between binding partner members (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described below.

"Percent (%) amino acid sequence homology " with respect to a reference polypeptide sequence is defined as the percentage of amino acid residues in a candidate sequence that are identical with the amino acid residues in the reference polypeptide sequence, after aligning the candidate sequence with the reference polypeptide sequence and introducing gaps if necessary to achieve maximum percent sequence homology and in the event that any conservative substitutions are not considered part of the sequence homology. Determination of percent amino acid sequence homology can be accomplished in a variety of ways in the art, for example, homology alignment can be performed using software such as BLAST, BLAST-2, ALIGN or Megalign (DNASTAR). Those skilled in the art can determine suitable parameters for aligning sequences, including any algorithms required to achieve maximal alignment over the full length of the sequences being compared.

For example, in the case of amino acid sequence comparison using ALIGN-2, the % amino acid sequence homology of a given amino acid sequence A to, with, or relative to a given amino acid sequence B is calculated as follows:

$$100 \times \text{fraction X/Y}$$

where X is the number of amino acid residues scored as identical matches by the sequence alignment program ALIGN-2 when the program aligns A with B, and where Y is the total number of amino acid residues in B. It will be understood that where the length of amino acid sequence A is not equal to the length of amino acid sequence B, the % amino acid sequence homology of A to B will not equal to the % amino acid sequence homology of B to A. All % amino acid sequence homology values used herein are obtained using the ALIGN-2 computer program unless otherwise indicated.

2. Antibody, preparation method, composition and article

1) Antibody

The present application relates to anti-CLDN18.2 antibodies. In certain embodiments, the present application provides an anti-CLDN18.2 antibody comprising at least 1, 2, 3, 4, 5 or 6 following hypervariable regions (HVRs) or binding domains referred to as complementarity determining regions (CDRs): (a) HVR-H1 comprising the amino acid sequence of the sequence SEQ ID NO:18 or SEQ ID NO:21, or an amino acid sequence having a homology of at least 90%, 95%, 96%, 97%, 98%, 99% to the sequence; (b) HVR-H2 comprising the amino acid sequence of sequence SEQ ID NO:19 or SEQ ID NO:22, or an amino acid sequence having a homology of at least 90%, 95%, 96%, 97%, 98%, 99% to the sequence; (c) HVR-H3 comprising the amino acid sequence of the sequence SEQ ID NO:20 or SEQ ID NO:23, or an amino acid sequence having a homology of at least 90%, 95%, 96%, 97%, 98%, 99% to the sequence; (d) HVR-L1 comprising the amino acid sequence of the sequence SEQ ID NO:24 or SEQ ID NO:27, or an amino acid sequence having a homology of at least 90%, 95%, 96%, 97%, 98%, 99% to the sequence; (e) HVR-L2 comprising the amino acid sequence of the sequence SEQ ID NO:25 or SEQ ID NO:28, or an amino acid sequence having a homology of at least 90%, 95%, 96%, 97%, 98%, 99% to the sequence; and (f) HVR-L3 comprising the amino acid sequence of SEQ ID NO:26 or SEQ ID NO:29, or an amino acid sequence having a homology of at least 90%, 95%, 96%, 97%, 98%, 99% to the sequence. In some cases, the heavy chain variable (VH) domain (region) of the anti-CLDN18.2 antibody may comprise an amino acid sequence having a homology of at least 90% (e.g., at least 91%, 92%, 93%, 94%, 95%, 96%, 97%, 98% or 99% sequence homology) to the sequence SEQ ID NO:36 or SEQ ID NO:37, or the amino acid sequence of SEQ ID NO:36 or SEQ ID NO:37, and/or its light chain variable (VL) domain (region) comprises an amino acid sequence having a homology of at least 90% (e.g., at least 91%, 92%, 93%, 94%, 95%, 96%, 97%, 98% or 99% sequence homology) to the sequence SEQ ID NO:38 or SEQ ID NO:39, or the amino acid sequence of SEQ ID NO:38 or SEQ ID NO:39.

In some embodiments, the anti-CLDN18.2 antibody comprises a heavy chain variable region and a light chain variable region, wherein the heavy chain variable region comprises the following amino acid sequence:

2) Antibody fragment

In certain embodiments, the antibodies provided herein are antibody fragments. Antibody fragments include, but are not limited to, Fab, Fab', Fab'-SH, (Fab')$_2$, Fv and scFv fragments and others described below. For a review of certain antibody fragments, see Hudson et al., Nat. Med. 9:129-134 (2003). For scFv fragments, see e.g., WO 93/16185.

A bifunctional antibody is an antibody fragment with two antigen-binding sites, which can be bivalent or bispecific. See e.g., EP 404,097; WO1993/01161. Trifunctional and tetrafunctional antibodies can be found in, e.g., Hudson et al., Nat. Med. 9:129-134 (2003).

A single domain antibody is an antibody fragment comprising all or part of a heavy chain variable domain or all or part of a light chain variable domain of an antibody. In certain embodiments, the single domain antibody is a human single domain antibody (see, e.g., US Patent No. 6,248,516 B1).

Antibody fragments can be produced by various techniques including, but not limited to, production by proteolytic digestion of intact antibodies and recombinant host cell (e.g., E. coli or phage).

3) Chimeric and humanized antibodies

In certain embodiments, the antibodies provided herein are chimeric antibodies. The preparation of chimeric antibodies can be found in, eg, US Patent No. 4,816,567.

In certain embodiments, the chimeric antibody is a humanized antibody. Typically, non-human antibodies are humanized to reduce immunogenicity to humans while retaining the specificity and affinity of the parental non-human antibodies. In general, humanized antibodies comprise one or more variable domains in which all HVR (CDR) regions, or portions thereof, are derived from non-human antibodies, and FRs (or portions thereof) are derived from human antibody sequences. A humanized antibody optionally comprises at least a portion of a human constant region. In some embodiments, some FR residues in a humanized antibody may be replaced with corresponding residues from a non-human antibody to repair or improve the affinity of the antibody.

For humanized antibodies and production methods thereof, see US Patent Nos. 5,821,337, 7,527,791, 6,982,321 and 7,087,409.

4) Human antibody

In certain embodiments, the antibodies provided herein are human antibodies. Human antibodies can be produced using various techniques known in the art.

Whole human antibodies or whole antibodies with human variable regions can be prepared by administering an immunogen to a modified transgenic animal, followed by challenge with the antigen. Such animals typically comprise all or part of the human immunoglobulin loci which replace the endogenous immunoglobulin loci or are present extrachromosomally or randomly integrated into the animal's chromosomes. In such transgenic mice, the endogenous immunoglobulin loci are generally inactivated. For methods of obtaining human antibodies from transgenic animals, see, e.g., US Patent Nos. 6,075,181 and 6,150,584 (describing XENOMOUSE™ technology); US Patent No. 5,770,429; US Patent No. 7,041,870 (describing K-M technology); and US application publication No. 2007/0061900. Human variable regions derived from intact antibodies produced by such animals can be further modified, e.g., by combining them with different human constant regions.

Human antibodies can also be made by hybridoma-based methods. Human myeloma and mouse-human hybrid myeloma cell strains for the production of human monoclonal antibodies have been described, see e.g., Boerner et al., J. Immunol., 147:86 (1991). Human antibodies produced via human B cell hybridoma technology are also described in Li et al., Proc. Natl. Acad. Sci. USA, 103: 3557-3562 (2006). Other methods include, e.g., those described in US Patent No. 7,189,826 (describing production of monoclonal human IgM antibodies from hybridoma cell strains) and Ni, Xiandai Mianyixue, 26(4):265-268 (2006) (describing human-human hybridomas).

Human antibodies can also be prepared by isolating Fv clone variable domain sequences selected from phage display libraries of human source. Such variable domain sequences can then be combined with the desired human constant domains.

Specifically, antibodies of the application with high affinity can be isolated by screening combinatorial libraries for antibodies with binding activity to CLDN18.2. For example, various methods are known in the art for generating phage display libraries and screening such libraries for antibodies with desired binding characteristics. Such methods can be found, for example, in Hoogenboom et al., Methods in Molecular Biology 178:1-37 (O'Brien et al., eds., Human Press, Totowa, NJ, 2001), Marks and Bradbury, Methods in Molecular Biology 248:161-175 (Edited by Lo, Human Press, Totowa, NJ, 2003) and Lee et al., J. Immunol. Methods 284(1-2): 119-132 (2004).

In some phage display methods, the VH and VL gene lineages are individually cloned by polymerase chain reaction (PCR) and randomly recombined in a phage library, followed by screening against antigen-binding phage. Phages typically present antibody fragments as single-chain Fv (scFv) fragments or Fab fragments. Patents describing human antibody phage libraries include, for example: US Patent No. 5,750,373 and US Patent Publication Nos. 2005/0079574, 2005/0119455, 2005/0266000, 2007/0117126, 2007/0237764, 2007/0292936 and 2009/0002360.

Antibodies or antibody fragments isolated from human antibody libraries are considered herein to be human antibodies or human antibody fragments.

5) Multispecific antibody

In any of the above aspects, the anti-CLDN18.2 antibodies provided herein are multispecific antibodies, e.g., bispecific antibodies. Multispecific antibodies are monoclonal antibodies that have binding specificities for at least two different sites. In certain embodiments, one binding specificity is for CLDN18.2 and the other binding specificity is for any other antigen (e.g., a second biomolecule, e.g., a cell surface antigen, e.g., a tumor antigen). Accordingly, bispecific anti-CLDN18.2 antibodies can have binding specificity for CLDN18.2 and tumor antigens such as CD3, CD20, FcRH5, HER2, LYPD1, LY6G6D, PMEL17, LY6E, CD19, CD33, CD22, CD79A, CD79B, EDAR, GFRA1, MRP4, RET, Steap1 or TenB2. Bispecific antibodies can be prepared as full-length antibodies or antibody fragments.

Techniques for making multispecific antibodies include, but are not limited to, recombinant co-expression of two immunoglobulin heavy chain-light chain pairs with different specificities, see WO 93/08829, WO2009/08025, and WO 2009/089004A1 etc.

6) Antibody variants

The antibodies of the present application encompass amino acid sequence variants of the anti-CLDN18.2 antibodies of the present application. For example, antibody variants prepared to further improve the binding affinity and/or other biological properties of the antibody may be desired. Amino acid sequence variants of an antibody can be prepared by introducing appropriate modifications into the nucleotide sequence encoding the antibody. Such modifications include, for example, deletions and/or insertions and/or substitutions of residues within the amino acid sequence of the antibody. Any combination of deletions, insertions and substitutions can be made to obtain the final construct, provided that the final construct has the desired characteristics, such as the binding property to the CLDN18.2 antigen.

In certain embodiments, antibody variants with one or more amino acid substitutions are provided. Substitution mutants (including conservative substitution mutants or non-conservative substitution mutants) can be obtained by substitution at one or more sites in the HVR (CDR) region and/or FR region.

Amino acids can be grouped according to common side chain properties:

(1) Hydrophobic: Norleucine, Met, Ala, Val, Leu, Ile;
(2) Uncharged and hydrophilic: Cys, Ser, Thr, Asn, Gln;
(3) Acidic: Asp, Glu;
(4) Basic: His, Lys, Arg;
(5) Residues affecting chain orientation: Gly, Pro;
(6) Aromatic: Trp, Tyr, Phe.

Conservative substitutions are defined as substitutions between the same group of amino acids, and non-conservative substitutions are defined as substitutions of amino acids from one of the different classes by amino acids from another class. Antibody variants of the application can be obtained by introducing amino acid substitutions into the antibodies of the application and screening the product for the desired activity (e.g., retention/improvement of antigen binding or improvement of ADCC or CDC).

The present application encompasses antibody variants obtained according to the antibodies disclosed herein that comprise non-conservative mutations and/or conservative mutations, so long as the variants still possess the desired CLDN18.2 binding activity.

One type of substitutional variants involves antibody variants that substitute one or more hypervariable region residues of a parent antibody (e.g., a humanized or human antibody). In general, the resulting variant selected for further study will be modified (e.g., improved) relative to the parent antibody with respect to certain biological properties (e.g., increased affinity) and/or will substantially retain certain biological properties of the parent antibody. Exemplary substitutional variants are affinity matured antibodies, which can be conveniently produced using, for example, phage display-based affinity maturation techniques such as those described herein. Briefly, one or more HVR (CDR) residues are mutated and the mutated antibody is displayed on phage, and the mutated antibody is screened for a particular biological activity (e.g., binding affinity).

In certain embodiments, substitutions, insertions or deletions may occur within one or more of the HVRs (CDRs), so long as such changes do not substantially impair the ability of the antibody to bind CLDN18.2. For example, conservative changes can be made in the HVR (CDR) that do not substantially reduce binding affinity. For example, such changes may be outside the antigen-contacting residues in the HVR, e.g., conservative or non-conservative amino acid substitutions may occur at one 1, 2, 3, 4, 5 amino acid residues of the FR region.

7) Recombination method

Anti-CLDN18.2 antibodies of the application can be prepared using recombinant methods, e.g., as described in US Patent No. 4,816,567. In one embodiment, an isolated nucleic acid encoding the anti-CLDN18.2 antibody described herein is provided. Such nucleic acids may encode the VL amino acid sequence and/or the VH amino acid sequence of the antibody. In another embodiment, one or more vectors (e.g., expression vectors) comprising such nucleic acids are provided. In another embodiment, host cells comprising such nucleic acids are provided. In one such embodiment, the host cell comprises (e.g., transformed to have): (1) a vector comprising a nucleic acid encoding an amino acid sequence comprising the VL of the antibody and an amino acid sequence comprising the VH of the antibody; or (2) a first vector comprising a nucleic acid encoding an amino acid sequence comprising the VL of the antibody and a second vector comprising a nucleic acid encoding an amino acid sequence comprising the VH of the antibody. In one embodiment, the host cell is a eukaryotic cell, such as a Chinese hamster ovary (CHO) cell or a lymphoid cell (e.g., Y0, NS0, Sp20 cells). In one embodiment, a method for making an anti-CLDN18.2 antibody is provided, wherein the method comprises culturing a host cell comprising a nucleic acid encoding the antibody as provided above under conditions suitable for expression of the antibody, and optionally recovering the antibody from the host cell (or host cell culture medium).

For recombinant production of anti-CLDN18.2 antibodies, nucleic acids encoding the antibody are isolated (e.g., as described above) and inserted into one or more vectors for further cloning and/or expression in host cells. Such nucleic acids can be readily isolated and sequenced using conventional procedures (e.g., by using oligonucleotide probes capable of specifically binding to the genes encoding the heavy and light chains of the antibody).

Suitable host cells for cloning or expressing antibody-encoding vectors include prokaryotic or eukaryotic cells as described herein. For example, antibodies can be produced in bacteria, especially when glycosylation and Fc effector functions are not required. For expression of antibody fragments and polypeptides in bacteria, see, e.g., US Patent Nos. 5,648,237, 5,789,199, and 5,840,523. Following expression, the antibody in the soluble fraction can be isolated from the bacterial cytoplasm and can be further purified.

In addition to prokaryotes, eukaryotic microorganisms such as filamentous fungi or yeast are also suitable cloning or expression hosts for antibody-encoding vectors, including fungal and yeast strains of which glycosylation pathways have been "humanized" to produce antibodies with partially or fully human glycosylation patterns. See Li et al., Nat. Biotech. 24: 210-215 (2006).

Host cells suitable for expression of glycosylated antibodies can also be derived from multicellular organisms (invertebrates and vertebrates). Examples of invertebrate cells include plant and insect cells. A number of baculovirus strains have been identified to be used for binding to insect cells, especially for transfection of Spodoptera frugiperda cells.

Plant cell cultures can also be used as hosts. See, e.g., US Patent Nos. 5,959,177, 6,040,498, 6,420,548, 7,125,978, and 6,417,429 (describing PLANTIBODIESTM technology for producing antibodies in transgenic plants).

Vertebrate cells can also be used as hosts. For example, mammalian cell lines suitable for growth in suspension may be suitable. Other examples of suitable mammalian host cell strains are SV40-transformed monkey kidney CV1 cell strains (COS-7); human embryonic kidney cell strains (e.g., 293 cells); baby hamster kidney cells (BHK); mouse sertoli cells (e.g., TM4 cells); monkey kidney cells (CV1); African green monkey kidney cells (VERO-76); human cervical carcinoma cells (HELA); canine kidney cells (MDCK); buffalo rat hepatocytes (BRL3A); human lung cells (W138); human hepatocytes (Hep G2); mouse breast tumors (MMT 060562); TRI cells; MRC 5 cells; Chinese hamster ovary (CHO) cells, including DHFR-CHO cells; and myeloma cell strains such as Y0, NSO and Sp2/0.

8) Immunoconjugate

The present application also provides immunoconjugates comprising the anti-CLDN18.2 antibody herein in combination with one or more cytotoxic agents, such as chemotherapeutic or chemotherapeutic drugs, growth inhibitors, toxins (for example, protein toxins, enzymatically active toxins of bacterial, fungal, plant or animal origin or fragments thereof) or radioisotopes.

In one embodiment, the immunoconjugate is an antibody-drug conjugate (ADC), wherein the antibody is conjugated to one or more drugs, including but not limited to maytansine, orlistatin, dolastatin, methotrexate, vindesine, taxane, trichothecene and CC1065.

In another embodiment, the immunoconjugate comprises a conjugate of the anti-CLDN18.2 antibody as described herein with an enzymatically active toxin, or a fragment thereof, including but not limited to diphtheria A chain, non-binding active fragments of diphtheria toxin, exotoxin A chain and trichothecene, etc.

In another embodiment, the immunoconjugate comprises a radio-conjugate formed by combining an anti-CLDN18.2 antibody as described herein with a radioactive atom. A variety of radioisotopes can be used to generate radio-conjugates. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$, and radioisotopes of Lu.

Conjugates of antibodies and cytotoxic agents can be made using a variety of bifunctional protein coupling agents, such as N-succinimidyl-3-(2-pyridyldithio)propionate (SPDP), succinimido-4-(N-maleimidomethyl)cyclohexane-1-carboxylate (SMCC), imidothiacyclopentane (IT), bifunctional derivatives of imidate ester (such as dimethyl adipate hydrochloride), active esters (such as disuccinimide octanedioate), aldehydes (such as glutaraldehyde), diazido compounds (such as bis (p-azidobenzoyl) hexanediamine), disazo derivatives (such as bis(p-diazobenzoyl) ethanediamine), diisocyanates (such as toluene 2,6-diisocyanate) and double active fluorine compounds (such as 1,5-difluoro-2,4-dinitrobenzene).

9) Pharmaceutical preparation

A pharmaceutical preparation of the anti-CLDN18.2 antibody herein is prepared by mixing the antibodies of the desired purity with one or more optional pharmaceutically acceptable carriers in the form of a lyophilized formulation or an aqueous solution. Pharmaceutically acceptable carriers are generally non-toxic to the acceptor at the dosages and concentrations employed, and include, but are not limited to: buffers, such as phosphates, citrates, and other organic acids; antioxidants, including ascorbic acid and methionine; preservatives (such as octadecyldimethylbenzyl ammonium chloride; hexahydrocarbon quaternary ammonium chloride; benzalkonium chloride; benzethonium chloride; phenol, butanol or benzyl alcohol; alkyl ethyl p-hydroxybenzoate such as methyl ethyl p-hydroxybenzoate or propyl ethyl p-hydroxybenzoate; catechol; resorcinol; cyclohexanol; 3-pentanol; and m-cresol); low molecular weight (less than about 10 residues) polypeptides; proteins such as serum albumin, gelatin or immunoglobulins; hydrophilic polymers such as polyvinylpyrrolidone; amino acids such as glycine, glutamic acid, asparagine, histidine, arginine or lysine; monosaccharides, disaccharides and other carbohydrates including glucose, mannose or dextrin; chelating agents such as EDTA; sugars such as sucrose, mannitol, fucose or sorbitol; salt-forming counterions, such as sodium; metal complexes (e.g., zinc-protein complexes); and/or nonionic surfactants, such as polyethylene glycol (PEG).

Exemplary lyophilized antibody formulations are described in US Patent No. 6,267,958. Aqueous antibody formulations include those described in US Patent No. 6,171,586 and WO2006/044908.

The preparation herein may also comprise more than one active ingredient as necessary for the particular indication being treated, preferably active ingredients having complementary activities that do not adversely affect each other. For example, it may be desirable to further provide additional therapeutic agents (e.g., chemotherapeutic agents, cytotoxic agents, growth inhibitors, and/or antihormone agents). Such active ingredients are suitably present in combination in amounts effective for the intended purpose.

10) Articles

In another aspect of the application, an article comprising the antibody or pharmaceutical composition of the application is provided. The article comprises a container and a label or package insert on or associated with the container. Suitable containers include, for example, bottles, vials, syringes, IV solution bags, and the like. Such containers may be formed from various materials, such as glass or plastic. The container holds the composition of the present application itself or in combination with another composition, and may have a sterile access port (e.g., the container may be an intravenous solution bag or a vial having a stopper which can be pierced by a hypodermic needle). At least one active agent in the composition is the antibody of the application. The label or package insert indicates that the composition is used to treat the selected tumor. Furthermore, the article can comprise (a) a first container comprising a composition therein, wherein the composition comprises an antibody of the application; and (b) a second container comprising a composition therein, wherein the composition comprises another tumor treatment drug or another antibody. The article in this embodiment of the application may further comprise a package insert indicating that such compositions can be used to treat tumors. Alternatively, or in addition, the article may further comprise a second (or third) container comprising a pharmaceutically acceptable buffer, such as bacteriostatic water for injection (BWFI), phosphate buffered saline, Ringer's solution and dextrose solution. It may further include other materials as may be desirable from a commercial and user standpoint, including other buffers, diluents, filters, needles and syringes.

11) Detection reagents and kits

The present application also provides cancer detection reagents comprising the anti-CLDN18.2 antibody or antigen-binding fragments thereof herein. Cancer detection reagents can be used to test tumor or blood tissue from actual patients. For example, testing can be performed using PCR, microarray or chip technology. Once cancer detection reagents are found to be present in a patient, those reagents can be selected for use in treating the patient or directing therapy.

In one embodiment, the anti-CLDN18.2 antibody or antigen-binding fragment thereof is chemically labeled, specifically, enzymatic labels such as alkaline phosphatase and glucose oxidase; fluorescent labels such as fluorescein and rhodamine; isotopes labels such as iodine (1251, 1311), carbon (14C), sulfur (35S), tritium (3H), indium (121In), and technetium (99mTc); or chemiluminescent labels such as luminol and fluorescein enzymes.

The present application also provides a cancer detection kit comprising the anti-CLDN18.2 antibody or antigen-binding fragment thereof herein. The kit may also comprise a labeled anti-CLDN18.2 antibody or antigen-binding fragment thereof. Specifically, the cancer detection kit can be used to detect gastric cancer, pancreatic cancer or esophageal cancer.

Specific embodiments of the present application will be described in more detail below with reference to the accompanying drawings. While specific embodiments of the present application are shown in the drawings, it should be understood that the present application may be embodied in various forms and should not be limited by the embodiments set forth herein. Rather, these embodiments are provided such that the present application will be more thoroughly understood, and will fully convey the scope of the present application to those skilled in the art.

**Example**

**Example 1 Preparation of materials for immunization and screening**

(1) Construction of human hCLDN18.2-TCE (T-cell epitope peptide) retrovirus expression plasmid

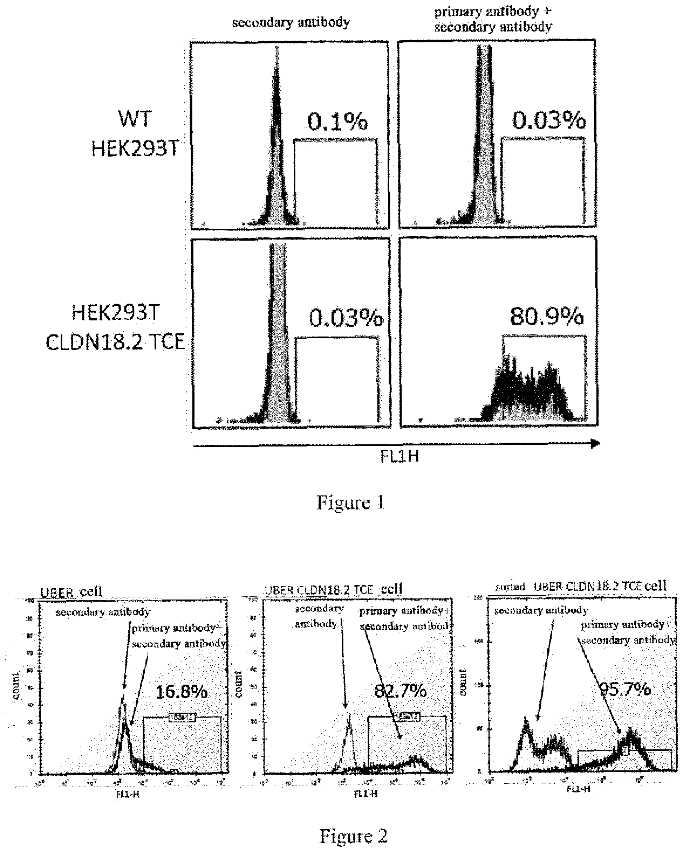

The hCLDN18.2 cDNA (SEQ ID NO:1) was cloned and fused with the T-cell epitope peptide at the C-terminal, resulting in hCLDN18.2-TCE. T-cell epitope peptides can make antigens break through immune tolerance and promote antibody production (Percival-Alwyn J. et al, mAbs, 2015, 7(1), 129-137). The hCLDN18.2-TCE was cloned into a retroviral vector to obtain the hCLDN18.2-TCE retroviral expression plasmid. The hCLDN18.2-TCE retrovirus expression plasmid was transiently transfected into HEK293-T cells, and the expression of hCLDN18.2 on the surface of HEK293-T cells was analyzed by flow cytometry 72 hours after transfection. The primary antibody (1st Ab) was the home-made positive control (Benchmarker) chimeric antibody 163E12. Cells were first incubated with the primary antibody (1 $\mu$g/mL) at 4°C for 45 min, washed with PBS, and then incubated with Alexa Fluor 488-labeled goat anti-human Fc secondary antibody (Thermofisher, Cat. No. A-11013)) (2nd Ab, 1:200 dilution) at 4°C for 45 minutes. Flow cytometry (FACS) analysis was performed after washing twice with PBS, and the negative control cells were incubated with secondary antibody only. The results were shown in Figure 1, showing that there was no hCLDN18.2 expression on the surface of wild HEK293-T cells; 80.9% of HEK293-T cells transiently transfected with hCLDN18.2-TCE retrovirus expression plasmid (HEK293T CLDN18.2 TCE) were positive cells expressing hCLDN18.2. This vector plasmid will be used in (1) DNA immunization scheme in Example 2.

(2) Mouse tumor cells expressing high levels of hCLDN18.2-TCE

The hCLDN18.2-TCE retrovirus expression plasmid was mixed with the lentiviral packaging plasmid and transfected into 293-T cells to prepare hCLDN18.2-TCE lentiviral particles. The hCLDN18.2-TCE lentiviral particles were transfected into mouse tumor cell lines, and 72 hours later, flow cytometry was performed and the mouse tumor cells pool with high expression of hCLDN18.2-TCE were detected and sorted. The primary antibody (1st Ab) was the home-made positive control (Benchmarker) chimeric antibody 163E12. Cells were first incubated with the primary antibody (1 $\mu$g/mL) at 4°C for 45 minutes, washed with PBS and then incubated with Alexa Fluor 488-labeled goat anti-human Fc secondary antibody (2nd Ab, 1:200 dilution) at 4°C for 45 minutes. Flow cytometry (FACS) analysis was performed after washing twice with PBS, and the negative control cells were incubated with the secondary antibody only. The results were shown in Figure 2, showing that only 16.8% of the wild-type mouse tumor cells (UBER parental) had positive signals on the surface and hCLDN18.2-TCE retrovirus-transfected mouse tumor cells (UBER CLDN18.2 TCE) had 82.7% positive cells. After sorting by flow cytometry analysis, up to 95.7% of mouse tumor cells had high levels of hCLDN18.2-TCE expression. The mouse tumor cells of hCLDN18.2-TCE will be used in (2) cellular immunization scheme in Example 2.

(3) Preparation of hCLDN18.2 extracellular loop 1 virus-like particles

According to the method published by Thorsten K. (Thorsten K., et al, Cancer Res, 2011, 71(2), 515-527), extracellular loop1 (ECL1) (SEQ ID NO:2) of hCLDN18.2 was inserted into the major immunodominant region (MIR) of Hepatitis B virus core antigen (HBcAg), and a G4SG4 linker was introduced at the N-terminal and C-terminal of ECL1, and a 6xHis tag was added to the C-terminal of the full-length fusion protein. The full-length gene of the fusion protein was synthesized, cloned into pET24a (+) plasmid, and then transfected into the BL21(DE3) E. coli expression system for the expression of the fusion protein. After purification of the fusion protein, HBV hCLDN18.2 extracellular loop 1 virus-like particles were prepared in renaturation buffer. The preparation of the extracellular loop 1 virus-like particles of hCLDN18.2 will be used in (3) virus-like particle multiple-point repetitive immunization-cellular immunization scheme and (4) virus-like particle tarsus joint immunization-cellular immunization scheme in Example 2.

(4) Construction of CLDN18 stably transfected cell strain

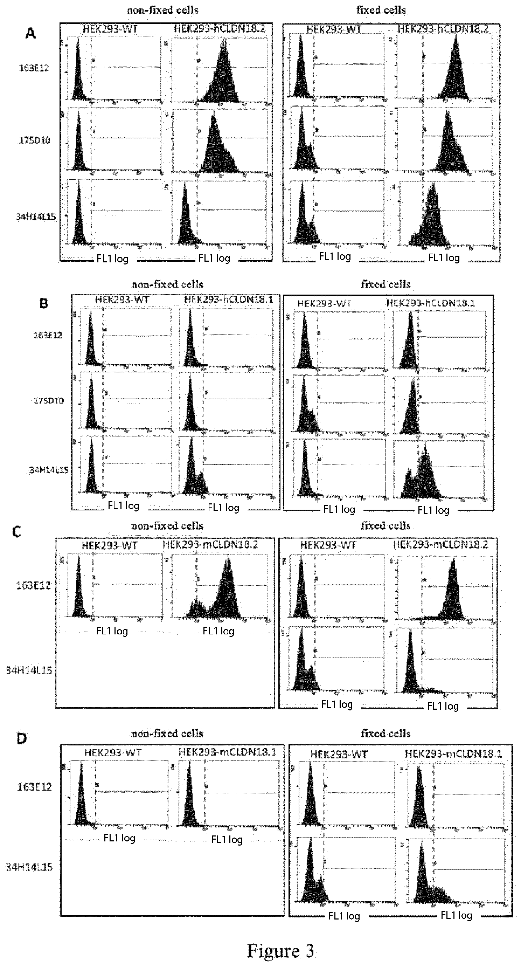

Full length hCLDN18.2 (SEQ ID NO:4), hCLDN18.1 (SEQ ID NO:5), mouse mhCLDN18.2 (SEQ ID NO:6), and mCLDN18.1 (SEQ ID NO:7) genes were synthesized respectively, and cloned into pcDNA3.4 vector plasmid respectively. Plasmids were transfected into HEK293 cells and neomycin (G418) was added for pressure selection. The Stably transfected HEK293 cell strains with high expression of hCLDN18.2, hCLDN18.1, m hCLDN18.2 and mCLDN18.1 were selected by limited dilution method. The test results of flow cytometry showed that the Stably transfected HEK293 cell strains (HEK293-hCLDN18.2, HEK293-mCLDN18.2) transfected with hCLDN18.2 and mCLDN18.2, both paraformaldehyde-fixed and non-fixed cells can be specifically recognized by home-made anti-CLDN18.2 positive control antibody 163E12 or 175D10 (Figure 3. A, C), and can also be recognized by the commercial broad spectrum anti-CLDN18 antibody 34H14L15 (Abcam catalog number ab203563) (Figure 3. A, C). Stably transfected HEK293 cell strains transfected with hCLDN18.1 and mCLDN18.1 (HEK293-hCLDN18.1, HEK293-mCLDN18.1), both paraformaldehyde-fixed and non-fixed cells cannot be specifically recognized by home-made anti-CLDN18.2 positive control antibody 163E12 or 175D10 (Figure 3. B, D), but can be recognized by the commercial broad spectrum anti-CLDN18 antibody (34H14L15) (Figure 3. B, D). The above Stably transfected HEK293 cell strains will be used for hybridoma screening in Example 3.

(5) Preparation of positive control antibodies 43A11, 175D10 and 163E12

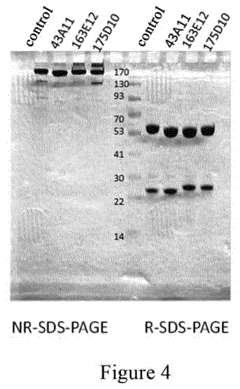

Human-mouse chimeric positive control antibodies 43A11 heavy chain (SEQ ID NO:8) and light chain (SEQ ID NO:9), 175D10 heavy chain (SEQ ID NO:10) and light chain (SEQ ID NO:11), 163E12 heavy chain (SEQ ID NO:12) and light chain (SEQ ID NO:13) full-length genes were synthesized respectively, with signal peptides added at the N-terminal respectively, and then cloned into pcDNA3.4 eukaryotic expression vector. The 43A11, 175D10 and 163E12 heavy chain and light chain expression plasmids were mixed and co-transfected into CHOS cells for transient protein expression. The supernatant was collected, affinity purified with Protein A and detected by SDS-PAGE. The results were shown in Figure 4.

**Example 2 Mouse immunization program and hybridoma preparation**

A total of 4 immunization schemes were adopted (Table 1), and at least two different strains of mice were used for each immunization scheme (Table 2). In each immunization scheme, some individual mice showed higher levels of serum immune titers. Mice individuals with high levels of immune titers in each immunization scheme were sacrificed and their spleens were removed. B lymphocytes were isolated, mixed, and then electrofused with cells from mouse myeloma cell lines to prepare hybridomas. A total of three batches of hybridoma preparation were prepared (Table 2).

Table 1

| Number | Immunization scheme | Immunization material | Mice strain and number | Immunization method and immune titer detection |
|---|---|---|---|---|
| 1 | DNA immunization scheme | CLDN18.2-TCE viral expression vector | NZB/W (M1, M2) BALC/c (M3, M4) | The CLDN18.2-TCE virus expression plasmid was injected through the tail vein for primary immunization (Zhang G F, et al. Human Gene Ther. 1999, 10, 1735-1737; Liu F, et al. Gene Ther. 1999, 6, 1258-1266), and the immune titer of the mouse serum was detected by flow cytometry three weeks after the immunization. The mice with serum reaction were boosted with HEK293 cells expressing high levels of human CLDN18.2 (Sotoshi N, et al. J Immunol Method, 2003, 280, 59-72). One week after booster immunization, flow cytometry was used to detect the immune titer of mouse serum. Mice with high immune titers were selected to be sacrificed and spleen B cells were fused to prepare hybridomas. |
| 2 | Cellular immunization scheme | Mouse tumor cell line expressing high levels of CLDN18.2-TCE | NZB/W (M1, M2, M3, M4) BALB/c (M1, M2, M3, M4) | Mice were subcutaneously inoculated with mouse tumor cell lines expressing high levels of CLDN18.2-TCE, and the immune titer of mouse serum was detected by flow cytometry in the second and fourth weeks. The mice with serum reaction and insignificant tumor growth were boosted at week 5 with mouse tumor cell lines expressing CLDN18.2-TCE (Sotoshi N, et al. J Immunol Method, 2003, 280, 59-72). And three days after booster immunization, mice with high immune titers were selected to be sacrificed and the spleen B cells were fused to prepare hybridomas. |
| 3 | Virus-like particle multiple-point repeat immunization-cellular immunization scheme | Virus-like particles fused to express extracellular loop 1 of CLDN18.2 | NZB/W (M1, M2) B6;129 (M3, M4) | Virus-like particles fused to express extracellular loop 1 of CLDN18.2 were first immunized by multiple-point repeat immunization method (Edward AG, Antibodies: A laboratory manual (Second Edition), 2012, Chapter 6, Protocol 30), and the immune titer of mouse serum against virus-like particles was detected by ELISA method one week later. The mice with high immune titer were selected for further immunization with the mouse tumor cell line with CLDN18.2-TCE, and the immune titer of the mouse serum was detected by flow cytometry at the fourth week after cell immunization. The mice with serum reaction were boosted with cells (Sotoshi N, et al. J Immunol Method, 2003, 280, 59-72), and three days later, the mice were sacrificed and spleen B cells were fused to prepare hybridomas. |

(continued)

| Number | Immunization scheme | Immunization material | Mice strain and number | Immunization method and immune titer detection |
|---|---|---|---|---|
| 4 | Virus-like particle tarsus joint immunization-cellular immunization scheme | Virus-like particles fused to express extracellular loop 1 of CLDN18.2 | NZB/W (M1, M2) B6;129 (M3, M4) SJL(M5, M6, M7) BALB/c (M8, M9, M10, M11) | Virus-like particles fused to express extracellular loop 1 of CLDN18.2 were preliminarily immunized by tarsus joint immunization method (Edward AG, Antibodies: A laboratory manual (Second Edition), 2012, Chapter 6, Protocol 18), and the immune titer of mouse serum against virus-like particles was detected by ELISA method one week later. The mice with high immune titer were selected for further immunization with the mouse tumor cell line with CLDN18.2-TCE, and the immune titer of the mouse serum was detected by flow cytometry at the fourth week after cell immunization. The mice with serum reaction were boosted with cells (Sotoshi N, et al. J Immunol Method, 2003, 280, 59-72), and three days later, the mice were sacrificed and spleen B cells were fused to prepare hybridomas. |

Table 2

| Mouse hybridoma preparation batch | Batch 1 | Batch 2 | Batch 3 |
|---|---|---|---|
| Source of mice with a high immune titer | DNA immunization scheme: BALB/c mouse M3, M4 | Virus-like particle multiple-point repeat immunization-cellular immunization scheme: NZB/W mouse M1, M2, B6; 129 mouse M3; <br><br> Virus-like particle tarsus joint immunization-cellular immunization scheme: B6;129 mouse M4, SJL mouse M6, BALB/c mouse M9 | Cellular immunization scheme: NZB/W mouse M3; <br><br> Virus-like particle multiple-point repeat immunization-cellular immunization scheme: <br><br> B6; 129 mouse M4; Virus-like particle tarsus joint immunization-cellular immunization scheme: B6; 129 mouse M3 |

**Example 3 Hybridoma Screening**

The hybridomas from three batches were cloned and cultured by limited dilution method, and the supernatant of the clone was used for three rounds of binding or reverse binding screening by flow cytometry, antibody typing and antibody-dependent cytotoxicity (ADCC) assay. A total of 128 hybridoma clones with ADCC function were screened out, which can specifically bind strongly to CLDN18.2 but not or weakly bind to CLDN18.1. The screening steps and the hybridoma clones obtained by screening at each stage were summarized in Table 3.

Table 3

| Mouse hybridoma batch | Batch 1 | Batch 2 | Batch 3 |
|---|---|---|---|
| Cloning by limited dilution | 10 3 84-well plates (plates 1-10), 0.8 cells/well | 10 3 84-well plates (plates 11-20), 4.8 cells/well (plates 11-15) and 3.4 cells/well (plates 16-20) | 10 3 84-well plates (plates 21-30), 1.3 cells/well |
| After 2 weeks of culture, the supernatant from the 384-well plate was taken for primary screening | | | |
| Primary screening | 341 positive clones (they were amplified, and cryopreserved, and the saturated supernatant was collected), MFI>100,000 | 336 positive clones, MFI>75,000 (plates 11-15), MFI>80,000 (plates 16-20) | 89 positive clones, MFI>90,000 |
| Confirmation screening | 165 positive clones, HEK293-hCLDN18.2 MFI>200,000, and HEK293-hCLDN18.1 MFI<100,000. (They were amplified, and cryopreserved, and the saturated supernatant was collected) | 120 positive clones, HEK293-hCLDN18.2 MFI>50,000, and HEK293-hCLDN18.1 MFI<40,000. (They were amplified, and cryopreserved, and the saturated supernatant was collected) | 21 positive clones, HEK293-hCLDN18.2 MFI>70,000, and HEK293-hCLDN18.1 MFI<30,000. (They were amplified, and cryopreserved, and the saturated supernatant was collected) |
| Verification screening | The supernatant of 306 positive clones derived from three confirmation screens were subjected to verification screening in 5 engineered cell lines (HEK293-hCLDN18.2 cells, HEK293-hCLDN18.1 cells, HEK293-mCLDN18.2 cells, HEK293-mCLDN18.1 cells and HEK293 wild-type cells) | | |
| Antibody typing | The supernatant of 306 positive clones derived from the three confirmation screens were subjected to antibody subtyping detection | | |
| Antibody-dependent cytotoxicity detection | Based on the results of the aforementioned experimental methods, the saturated supernatant of 128 positive clones were selected for antibody-dependent cytotoxicity detection. | | |
| The supernatant of 128 positive clones was subjected to immunohistochemistry (IHC) detection on FFPE tissue sections | | | |

1) Primary screening: binding of the expression product in the supernatant of each clone to a mouse tumor cell line expressing high levels of CLDN18.2-TCE (UBER CLDN18.2 TCE) was analyzed by flow cytometry. The fused hybridomas were inoculated into 384-well plates by the limited dilution method, and the supernatant was collected after ten days of culture to screen positive hybridoma clones by flow cytometry. After co-incubating the supernatant with UBER CLDN18.2 TCE cells (20,000 cells/well) at 4°C for 45 minutes, the plate was washed. Alexa Fluor-488-goat anti-mouse IgG (ThermoFisher Catalog No. A28175) was used as the secondary antibody for flow cytometry detection, and clones with an average fluorescence intensity of above 100,000 were screened for amplification, and the saturated supernatant was collected for confirmation screening.

3 batches of hybridomas were cloned by using the limited dilution method in 10 384-well plates per batch. After primary screening, 341 (MFI>100,000), 336 (MFI>75,000 or MFI>80,000) and 89 (MFI>90,000) hybridoma clones that positively bound to the mouse tumor cell line expressing CLDN18.2-TCE, were obtained from three batches of hybridomas, respectively.

2) Confirmation screening: the supernatant expression products of the positive clones screened in the previous step were incubated with HEK293-hCLDN18.2 cells, HEK293-hCLDN18.1 cells and HEK293 wild-type cells, respectively (20,000 cells/well, 4°C, 45 minutes). Clones positive for binding to HEK293-hCLDN18.2 cells but negative for binding to HEK293-hCLDN18.1 cells and HEK293 wild-type cells were screened by flow cytometry using Alexa Fluor-488-goat anti-mouse IgG (ThermoFisher Cat. No. A28175) as secondary antibody for amplification, and the saturated supernatant was collected for validation screening.

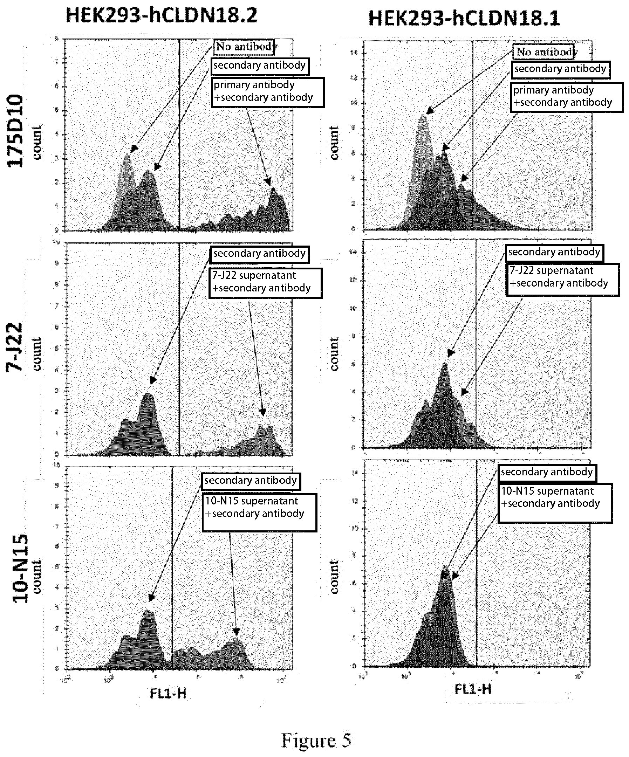

After confirmation screening, 165 (HEK293-hCLDN18.2 MFI>200,000, HEK293-hCLDN18.1 MFI<100,000), 120 (HEK293-hCLDN18.2 MFI>50,000, HEK293-hCLDN18.1 MFI<40,000) and 21 (HEK293-hCLDN18.2 MFI>70,000, HEK293-hCLDN18.1 MFI<30,000) clones that can specifically bind to HEK293-hCLDN18.2 cells strongly but bind to HEK293-hCLDN18.1 cells weakly and does not bind to HEK293 wild-type cells, were obtained from three batches of hybridomas, respectively. FACS analysis of some positive clones was shown in Figure 5.

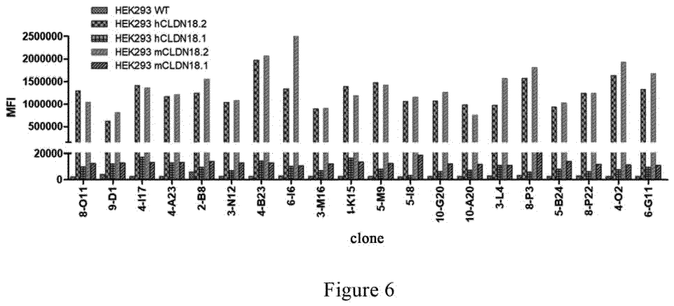

3) Verification screening: the supernatant expression products of 306 (165 + 120 + 21) positive hybridomas obtained in the confirmation screening of three batches of hybridomas were incubated with HEK293-hCLDN18.2 cells, HEK293-hCLDN18.1 cells, HEK293-mCLDN18.2 cells, HEK293-mCLDN18.1 cells, and HEK293 wild-type cells (20,000 cells/well, 4°C, 45 minutes), and Alexa Fluor-488-goat anti-mouse IgG (ThermoFisher Cat. No. A28175) was used as a secondary antibody for analysis by flow cytometry. The flow cytometry analysis results of some clones on the 5 cell lines were shown in Figure 6, showing that the mean fluorescence intensity (MFI) of positive clones in HEK293-hCLDN18.2 cells and HEK293-mCLDN18.2 cells exceeds 500,000, while the mean fluorescence intensity in HEK293-hCLDN18.1 cells, HEK293-mCLDN18.1 cells and HEK293 wild-type cell lines was all below 50,000, and the mean fluorescence intensity (MFI) of most clones (16/22) in HEK293-hCLDN18.2 cells and HEK293-mCLDN18.2 cells exceeds that in HEK293-hCLDN18.1 cells and HEK293-mCLDN18.1 cells 50 times.

4) Antibody typing: because different subtypes of IgG molecules also had different abilities to mediate ADCC effects. The antibodies of the expression products in the supernatant derived from 306 (165+120+21) positive hybridomas obtained in the three batches of hybridoma confirmation screening were subjected to mouse antibody subtyping using a mouse antibody subtype detection kit prior to antibody-dependent cell killing assay. Most of the 306 clones belong to mouse IgG2a, IgG2b or IgG3 with ADCC function, and only a few (26/306) were IgG1 subtype without ADCC function (corresponding to human antibody IgG4 subtype). Some clones were mixed subtypes, possibly because the hybridoma clones were not monoclonal. The light chains were all mouse kappa isoforms.

5) ADCC effect detection

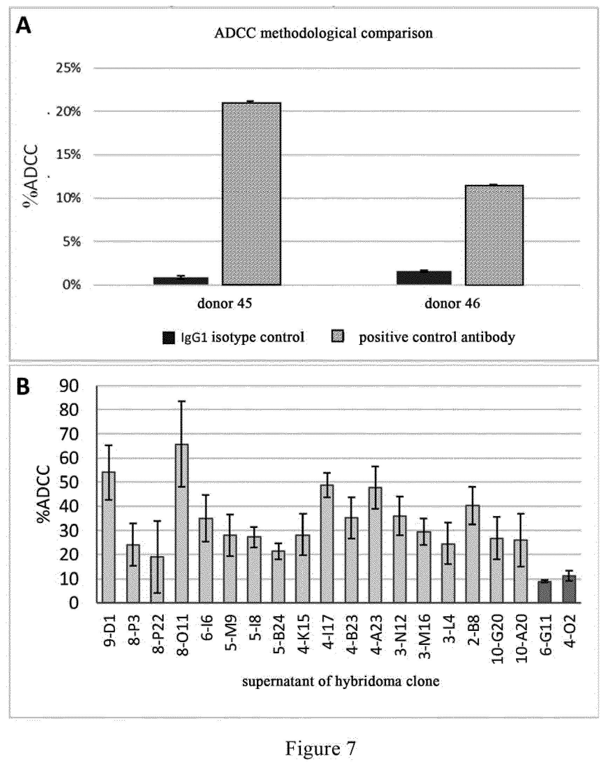

Taking into account the aforementioned confirmation screening, verification screening and antibody typing results, the saturated supernatants of 128 positive clones were selected for antibody-dependent cytotoxicity function detection. The effector cells were human peripheral blood mononuclear cells (PBMCs), which were derived from two donors (Nos. 45 and 46). Blood was drawn from the donors the day before the experiment. The collected blood was stored at room temperature, and the PBMCs were freshly isolated through the Ficoll gradient. The target cells were HEK293 cells expressing CLDN18.2 (HEK293-hCLDN18.2). The hybridoma expression products were uniformly diluted 4-fold. Freshly obtained PBMCs and target cells were incubated with each sample for 4 hours at an effect-to-target ratio of 25:1. ADCC effect of the antibody was characterized by measuring lactate dehydrogenase (LDH) associated with cytotoxicity. The absorbance value detected when the target cells were lysed spontaneously was defined as 0%, and the absorbance value detected when the target cells were completely lysed was defined as 100%, and the ADCC effect was characterized by the relative percentage activity of each test sample. The ADCC effect results of some clones were shown in Figure 7. The ADCC killing effect of the clone supernatant was higher than 20% on average, the ADCC effect of some clones (9D-1, 8-011) was higher than 50%, and the ADCC effect was less than 10% for mouse IgG1 subtypes 6-G11 and 4-O2.

**Example 4 Screening of hybridomas with positive immunohistochemical staining for hCLDN18.2 on PPFE tissue sections**

In the process of companion diagnosis, pathological detection of paraffin-embedded (FFPE) histological sections after biopsy of the patient's tumor tissue was a common procedure. Tumor associated antigen (TAA) loses its natural structure in the process of histological sectioning, and even if antigen retrieval was performed, the antigen can only restore part of the conformational structure to a certain extent, or only expose certain linear peptides. The supernatant of the above-mentioned preferred 128 positive hybridoma clones had the ability to specifically bind to the native CLDN18.2 conformation on the cell surface and ADCC function, but as a companion diagnostic antibody, IHC screening for antigen-specific binding must be performed on FFPE tissue sections. In this example, binding of the supernatant of 128 positive hybridoma on cell block paraffin-embedded (FFPE) tissue sections of 3 engineered cell lines (HEK293-hCLDN18.2 cells, HEK293-hCLDN18.1 cells, and HEK293 wild-type cells) was detected by immunohistology chemistry (IHC) method of PPFE tissue sections.

First, IHC staining was performed on FFPE tissue sections of HEK293 cell blocks stably expressing CLDN18, including culturing HEK293-hCLDN18.2 cells, HEK293-hCLDN18.1 cells and HEK293 wild-type cells, collecting cells, centrifuging to form cell blocks, then performing dehydration, clearing, and paraffin-embedding steps followed by FFPE tissue sectioning and IHC staining. The supernatant of hybridoma clones that can be positively stained on HEK293-hCLDN18.2 cell block FFPE tissue sections and negatively stained on HEK293-hCLDN18.1 cells and HEK293 wild-type cell block FFPE tissue sections was screened. Then, the obtained positive hybridoma supernatant was verified by IHC staining on FFPE tissue sections of human gastric cancer tissue. Finally, IHC staining was performed with the verified IHC-positive hybridoma supernatant on human normal stomach and gastric cancer FFPE tissue micro-array (TMA). The final IHC-positive colonies were determined according to the IHC staining intensity and staining ratio.

The IHC method of hybridoma clones for cell block FFPE tissue sections, gastric cancer FFPE tissue sections, and normal stomach and gastric cancer FFPE tissue micro-array (TMA) includes the following steps: 1) slide heating for deparaffinization: 60°C, 30 minutes; 2) deparaffinizing: immersion in three stages (I, II, III) of xylene, 5 minutes per stage; 3) hydration: immersion in gradient ethanol 100% × 2 times, 95% × 1 time, 75% × 1 time, 3 minutes each time, and immersion in distilled water for 5 minutes; 4) antigen retrieval: the antigen retrieval solution (Tris-EDTA, pH=9.0) was put in a water bath at 100°C, and the FFPE tissue sections were put in the antigen retrieval solution, incubated for 30 minutes, cooled at room temperature for 30 minutes, and washed 3 times with PBS buffer for 5 minutes each time; 5) blocking: incubated in 3% H2O2-PBS for 10 minutes at room temperature in the dark, washed 3 times with PBS, then incubated in 5% BSA (PBS-BSA) for 60 minutes at room temperature, and washed 3 times with PBS buffer; 6) primary antibody incubation: commercial rabbit anti-CLDN18.2 positive antibody (EPR19202, Abeam, Cat. No. ab222512) diluted 1:100, or mouse hybridoma supernatant, incubated overnight at 4°C; 7) secondary antibody incubation: HRP-conjugated goat anti-rabbit (Abcam, catalog number ab6721, 1:1000 dilution) and HRP-conjugated goat anti-mouse secondary antibody (ThermoFisher, catalog number 62-6520, 1:2000 dilution); 8) staining: staining with diaminobenzidine (DAB) for 3 minutes; 9) counterstaining with hematoxylin for 1 minute, differentiation with 0.5% HCl-75% ethanol for several seconds, and bluing with running water for 20 minutes; 10) dehydration and clearing: dehydration with 70% ethanol, 80% ethanol, 95% ethanol, anhydrous ethanol I and anhydrous ethanol for 2 minutes each; clearing with Xylene I and Xylene II for 5 minutes each; 11) mounting: mounting with a neutral mounting medium.

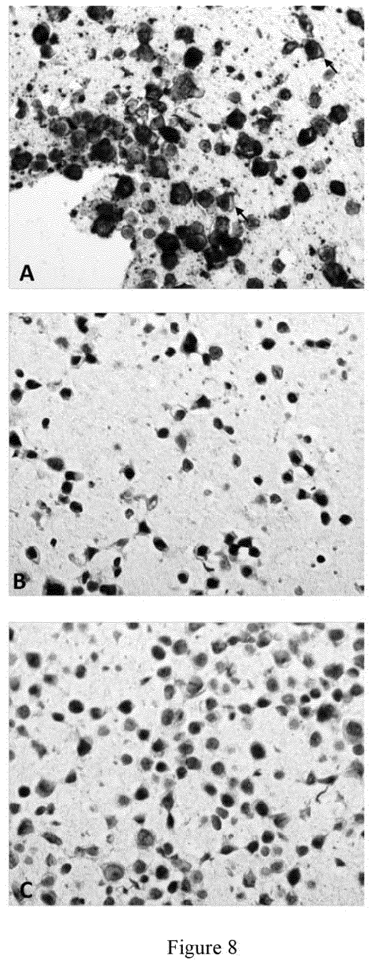

Figure 8 showed the IHC staining results of the positive control commercial antibody EPR19202 on FFPE tissue sections of the HEK293 cell block, wherein the antibody can specifically stain FFPE tissue sections of the cell blocks of the HEK293-hCLDN18.2 stable cell line, and the positive staining was obvious at the location of the cell membrane (Figure 8. A, indicated by the arrow), which was consistent with the conclusion that CLDN18.2 was a membrane protein (Figure 8A). In contrast, the antibody showed no obvious staining on the FFPE tissue sections of the cell blocks of the stable HEK293-hCLDN18.1 cell line (Figure 8B), and no obvious staining on the HEK293 wild cell line (Figure 8C).

4 Hybridomas clones (5-15, 5-110, 5-G17 and 10-D3) were obtained by IHC staining of the supernatant of 120 hybridoma clones on FFPE tissue sections of cell blocks of the 3 cell lines (HEK293-hCLDN18.2 cells, HEK293-hCLDN18.1 cells and HEK293 wild-type cells). The supernatant of the clones shows positive IHC staining on FFPE tissue sections of the HEK293-hCLDN18.2 cell block, while showing no or weak staining on FFPE tissue sections of the HEK293-hCLDN18.1 and HEK293-WT cell blocks.

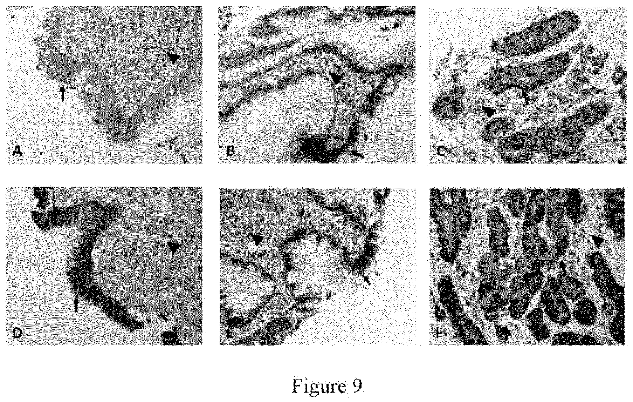

The supernatant of 4 IHC-positive hybridoma clones screened using FFPE tissue sections of the HEK293-hCLDN18.2 cell block was verified by IHC staining on FFPE tissue sections derived from the human gastric cancer tissues, wherein the IHC staining results of the supernatant of hybridoma clone 5-15 on FFPE tissue sections of 3 cases of human gastric cancer tissues were shown in Figure 9 (A, B, C). The positive staining of the antibody only appears at the location of the glandular epithelial cells (indicated by the arrow), and there was no obvious positive chromogenic reaction or weak staining for stromal cells (indicated by triangular arrows). The positive staining positions and the positive staining tissue structure of 5-15 on FFPE tissue sections of the 3 cases of gastric cancer tissues were relatively consistent with the IHC staining results of the positive control antibody (EPR19202) (Figures 9D, E and F).

As a companion diagnostic antibody, it was of great clinical significance to recognize a high proportion of patients with high expression of tumor-associated antigens for treatment. Because of the differences of cancer patients and the heterogeneity of tumor tissues of patients, the IHC staining results of different diagnostic antibodies against the same tumor-associated antigen were often different. We performed IHC staining of the supernatant with expression products of the 4 IHC positive candidate hybridomas and the mixture of the supernatant from the 4 (Cocktail) on the FFPE tissue microarray (TMA) chips (Biomax, ST807) of the human normal stomach and gastric cancer FFPE tissues. During antibody incubation, the incubation time of the supernatant of single hybridoma clone and positive control antibody was 30 minutes, and the incubation time of the supernatant mixture of 4 hybridomas was 120 minutes. Table 4 summarized the tissue sites and tissue types of membrane-positive staining and cytoplasmic-positive staining, and the corresponding positively stained clones. 5-110, 5-G17, 5-15, 10-D3 and the mixture of supernatant of the 4 clones showed cell membrane positive staining rates of 28.9%, 17.1%, 14.5%, 11.8% and 28.9% on all 76 tissue sites, respectively, and the cell membrane positive rate of the positive control antibody (EPR19202) was 23.7%. It was worth noting that almost all clone supernatant and positive antibodies were positively stained on normal gastric tissues (5-110 87.5%, 5-G17 87.5%, 5-15 100%, 10-D3 87.5%, Mix. Of 4 100%, EPR19202 100%), which was consistent with the specific expression of CLDN18.2 in normal gastric mucosa epithelial cells reported in the literature (Sahin. U. et al, Human Cancer Biology, 2008, 14(23), 7624-7634). Compared with the high positive rate staining of normal gastric tissue, the positive staining rate of each hybridoma supernatant on the gastric cancer tissue varies greatly, the cell membrane positive staining rates on gastric cancer tissues were 23.5% for 5-110, 10% for 5-G17, 4.4% for 5-15, 2.9% for 10-D3 and 22% for the mixture of supernatant of the 4 hybridomas (Mix. Of 4), which may be related to the high heterogeneity of the gastric cancer tissue. Among them, the supernatant of 5-110 hybridoma clone had the highest positive staining rate in the gastric cancer tissue, reaching 23.5%, which was significantly higher than 14.7% of the positive control antibody EPR19202, and was substantially consistent with the positive rate of 16-23% reported by Zhu G et al (Zhu G et al, Sci Rep., 2019, 9: 8420-8431).

EP 4 105 237 A1

It should be particularly noted that the positive staining rates of 5-110 and 5-G17 in the gastric cancer FFPE tissue was high, and the composite (5-110 + 5-G17) positive rate of the two reached up to 26%. The gastric cancer FFPE tissue sites with positive staining sites substantially include the positive sites appeared on 5-15 and 10-D3. Therefore, using recombinantly expressed and purified antibodies 5-110 and 5-G17 in a certain proportion to form an antibody mixture for companion diagnosis can maximize the selection of gastric cancer patients with high CLDN18.2 expression, which was significant for guiding the clinical administration of drugs targeting CLDN18.2.

Table 4

| Position | Number | Diagnostic type | Mixture of 4 antibodies | 5-I10 | 5-G17 | 5-I5 | 10-D3 | EPR19202 |
|---|---|---|---|---|---|---|---|---|
| A6 | 6 | Gastric adenocarcinoma | M | M | M | M | M | M |
| H3 | 73 | Normal stomach tissue | M | M | M | M | M | M |
| H4 | 74 | Normal stomach tissue | M | M | M | M | M | M |
| H7 | 77 | Normal stomach tissue | M | M | M | M | M | M |
| H8 | 78 | Normal stomach tissue | M | M | M | M | M | M |
| H9 | 79 | Normal stomach tissue | M | M | M | M | M | M |
| H10 | 80 | Normal stomach tissue | M | M | M | M | C | M |
| F9 | 59 | Gastric adenocarcinoma | M | M | M | M | / | M |
| H6 | 76 | Normal stomach tissue | M | M | C | M | M | M |
| E2 | 42 | Gastric adenocarcinoma | M | M | M | C | / | / |
| F4 | 54 | Gastric adenocarcinoma | M | M | C | C | / | M |
| F1 | 51 | Gastric adenocarcinoma | M | M | C | C | / | C |
| B6 | 16 | Gastric adenocarcinoma | M | M | C | C | / | / |
| C11 | 21 | Gastric adenocarcinoma | M | M | / | / | / | M |
| B2 | 12 | Gastric adenocarcinoma | M | M | / | / | / | / |
| B3 | 13 | Gastric adenocarcinoma | M | M | / | / | / | / |
| C2 | 22 | Gastric adenocarcinoma | M | M | / | / | / | / |
| F10 | 60 | Gastric adenosquamous carcinoma | M | M | / | / | / | / |
| H5 | 75 | Normal stomach tissue | M | C | C | M | M | M |
| B7 | 17 | Gastric adenocarcinoma | M | C | M | C | / | / |
| C3 | 23 | Gastric adenocarcinoma | M | C | / | C | / | / |
| E4 | 44 | Gastric adenocarcinoma | M | / | / | / | / | / |
| C9 | 29 | Gastric adenocarcinoma | C | M | C | C | / | M |
| E1 | 41 | Gastric adenocarcinoma | C | M | / | C | / | / |
| C6 | 26 | Gastric adenocarcinoma | C | M | M | / | / | M |
| B10 | 20 | Gastric adenocarcinoma | C | M | / | / | / | / |
| A7 | 7 | Gastric adenocarcinoma | C | C | M | M | / | M |
| B8 | 18 | Gastric adenocarcinoma | C | C | M | C | / | / |
| A4 | 4 | Gastric adenocarcinoma | C | / | C | C | M | M |
| A5 | 5 | Gastric adenocarcinoma | C | C | C | C | C | M |
| D9 | 39 | Gastric adenocarcinoma | C | C | C | C | C | M |

(continued)

| Position | Number | Diagnostic type | Mixture of 4 antibodies | 5-I10 | 5-G17 | 5-I5 | 10-D3 | EPR19202 |
|---|---|---|---|---|---|---|---|---|
| Total cell membrane positive staining rate | | | 22/76 (28.9%) | 22/76 (28.9%) | 13/76 (17.1%) | 11/76 (14.5 %) | 9/76 (11.8%) | 18/76 (23.7%) |

| Summary of tissue microarray IHC results | | | | Mixture of 4 antibodies | 5-I10 + 5-G17 mixture | 5-I10 | 5-G17 | EPR19202 |
|---|---|---|---|---|---|---|---|---|
| Cell positive rate (%) | membrane staining | Normal stomach tissue | | 8/8 (100%) | 7/8 (87.5%) | 7/8 (87.5%) | 7/8 (87.5%) | 8/8 (100%) |
| | | Gastric cancer tissue | | 15/68 (22%) | 18/68 (26%) | 16/68 (23.5%) | 7/68 (10%) | 10/68 (14.7%) |

Note: 1) the table counted the tissue sites with positive staining; 2) TMA had a total of 80 tissue sites, of which 4 tissue sites had poor tissue morphology and were excluded; 3) M indicated cell membrane positive staining, and C indicates cytoplasm positive staining, and / indicated negative staining.

EP 4 105 237 A1

**Example 5. Subcloning of hybridoma clones with hCLDN18.2 positive staining FFPE tissue sections and sequencing for the antibody molecules**

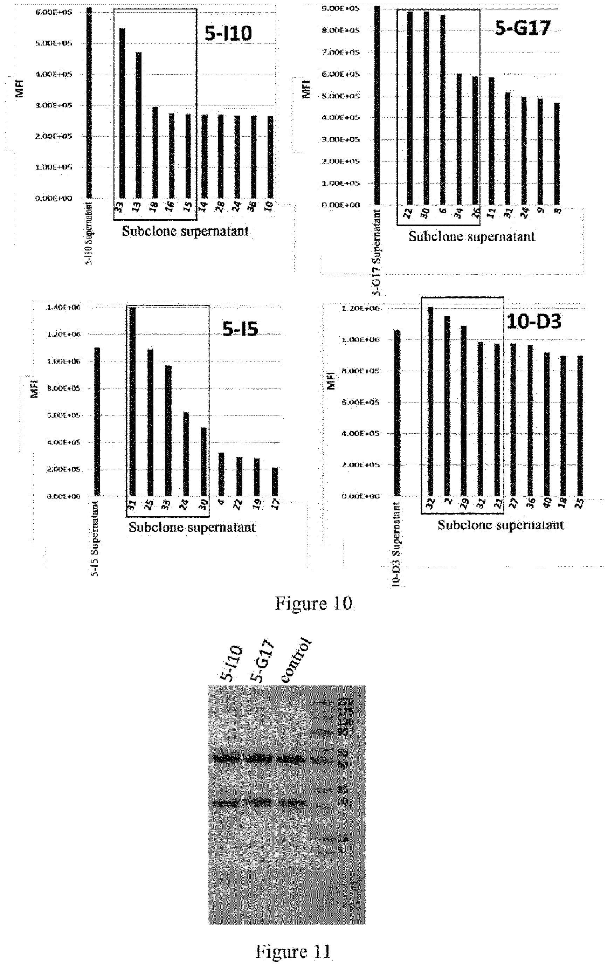

In this example, the limited dilution method was used for subcloning of the 4 hybridoma clones (5-110, 5-G17, 5-15 and 10-D3) with IHC-positive FFPE tissue sections and sequencing of the variable regions of the antibody molecules was performed. The thawed hybridoma clones were subjected to limited dilution and plated into 192-well plates at a density of 0.8 cell/well. Single clones were selected by microscopy, and the supernatant were collected after 14 days of culture. The specific binding of the subclone supernatant to HEK293-hCLDN18.2 cells was detected by flow cytometry analysis, and meanwhile the single clone was amplified and cultured. At the same time, single clones were amplified and cultured. The flow cytometric analysis method was as follows: after co-incubating the monoclonal supernatant with HEK293-CLDN18.2 cells (20,000 cells/well) at 4°C for 45 minutes, the plate was washed, and Alexa Fluor-488-goat anti-mouse IgG (ThermoFisher, Catalog No. A28175) was used as a secondary antibody for flow cytometry detection. For each hybridoma, 10 (5-15, 9) single clones were selected for amplification and cryopreservation, and the supernatant was collected for IHC staining verification on FFPE tissue sections. The flow cytometry analysis of HEK293-hCLDN18.2 cells using the monoclonal supernatant of 4 hybridomas was shown in Figure 10, showing that the monoclonal supernatant maintains the specific binding to HEK293-hCLDN18.2 cells, and the binding intensity of part of the monoclonal supernatant to HEK293-hCLDN18.2 cells was comparable to that of the corresponding hybridoma supernatant (Parental supe.). The top 5 monoclonal supernatant (shown in blue box in Figure 9) with the highest binding intensity to HEK293-hCLDN18.2 cells were selected from each hybridoma clone for IHC verification of FFPE tissue sections, and all monoclonal supernatant showed IHC positive staining. The 2 single clones with the highest IHC intensity on FFPE tissue sections were selected for each hybridoma (5-15-31 and 5-15-25, 10-D3-22 and 10-D3-21, 5-G17-30 and 5-G17-6, 5-110-33 and 5-110-18) for gene clone sequencing of the heavy and light chain variable regions.

The VH and VL DNA sequences of the hybridoma single clones were amplified using the RACE (rapid amplification of cDNA ends) method. RNA was extracted from amplified hybridoma monoclonal cells, which was reverse transcribed into cDNA and subjected to a 5'-RACE reaction using a combination of 5'-universal primers and 3'-H, L(κ) or L(λ) FR1 degenerate primers to perform PCR for the heavy chain or light chain V region, and the PCR-amplified fragments were ligated to a sequencing vector by the TOPO clone method for sequencing. Sequencing results showed that 5-15-31 and 5-15-25 had the same VH sequences, but different VL sequences, indicating that 5-15-31 and 5-15-25 were different single clones. Except for 5-15, the two single clones sequenced for each of 10-D3, 5-G17 and 5-110 had identical VH and VL, wherein the VH and VL DNA sequences of 5-110 and 5-G17 were shown in Table 5, and the corresponding amino acid sequences were shown in Table 6, wherein the CDR regions in the light and heavy chains were divided according to the Chothia numbering system.

Table 5

| Antibody name | DNA sequences of the heavy chain V region |
| --- | --- |
| 5-I10 | GAGATCCAGCTGCAGCAGTCTGGAGCTGAGCTGGTGAAGCCTGGGGCTT CAGTGAAGATATCCTGCAAGGCTCCTGGTTACTCATTCACTGACTACAATA TGAACTGGGTGAAGCAGAGCCATGGAAAGAGCCTTGAGTGGATTGGAAA TATTAATTCTTACTATGGTAGTACTAGCTACAATCAGAAGTTCAAGGGCAA GGCCACATTGACTGTAGACAGATCTTCCAGCACAGCCTACATGCAGCTCA ACAGCCTGACATCTGAGGACTCTGCAGTCTATTACTGTACAAACTTCGAC AGGGGAAATTCGTTCCCTTACTGGGGCCACGGGACTCTGGTCACTGTCTC TGCA(SEQ ID NO:14) |
| 5-G17 | GAGGTGAAACTGGTGGAGTCTGGGGGAGGCTTAGTGAAGCCTGGAGGG TCCCTGAAACTCTCCTGTGCAGCCTCTGGATTCACTATCAGTGACTACGG AATGGCGTGGGTTCGACAGGCTCCAGGGAAGGGGCCTGAGTGGGTAGCA TTCATTAGTAATTTGGCATATAGTATCCACTATGTAGACACTGTGACGGGC CGATTCACCATCTCTAGAGAGAATGCCAAGAACACCCTGTACCTGGAAAT GCGTAGTCTGAGGTCTGAGGACACAGCCATGTATTACTGTGCAAGAATGT ACTATGGGAATGCTTTGGACTACTGGGGTCAAGGAACCTCAGTCACCGTC TCCTCA(SEQ ID NO:15) |

(continued)

| Antibody name | DNA sequences of the light chain V region |
|---|---|
| 5-I10 | GACATTGTGATGACACAGTCTCCATCCTCCCTGACTGTGACAGTAGGAGA GAAGGTCACTATGACCTGCAAGTCCAGTCAGAGTCTGTTAAACAGTGGA AATCAAAAGAACTATTTGACCTGGTACCAGCAGAAACCAGGGCAGTCTC CTAAACTGTTGATCTACTGGGCATCCACTAGGGAATCTGGGGTCCCTGAG CGCTTCACAGGCAGAGGATCTGGAACAGATTTCACTCTCACCATCAGCA GTGTGCAGGCTGAAGACCTGGCAGTTTATTACTGTCAGAATGGTTATAGT TATCCATTCACGTTCGGCTCGGGGACAAACTTGGAAATAAAA(SEQ ID NO:16) |
| 5-G17 | GACATTGTGATGACACAGTCTCCATCCTCCCTGACTGTGACAACAGGAGA GAAGGTCACTATGAGTTGCAAGTCCAGTCAGAATCTGTTAAACAGTGGA AATCAAAAGAACTACTTGACCTGGTACCAGCAGAAACCAGGGCAGCCTC CTAAACTGTTGATCTACTGGGCATCCACTAGGGAATCTGGGGTCCCTGAT CGCTTCACAGGCAGTGGATCTGGAACAGATTTCACTCTCACCATCAGCAG TGTGCAGGCTGAAGACCTGGCAGTTTATTACTGTCAGAATGATTATAGTTT TCCTCTCACGTTCGGTGCTGGGACCAAGCTGGAGCTGAAA(SEQ ID NO:17) |

Table 6 Amino acid sequences of CDRs

| Heavy chain CDR (Chothia numbering) | | | | | | | |
|---|---|---|---|---|---|---|---|
| Antibody name | FR1 | CDR1 | FR2 | CDR2 | FR3 | CDR3 | FR4 |
| 5-I10 (heavy chain variable region: SEQ ID NO: 36) | EIQLQQSGA ELVKPGASV KISCKAP(SE Q ID NO:40) | GYSFTD Y(SEQ ID NO:18) | NMNWVKQ SHGKSLEWI GNI(SEQ ID NO:41) | NSYYGS ( SEQ ID NO:19) | TSYNQKFKGKAT LTVDRSSSTAYM QLNSLTSEDSAV YYCTN(SEQ ID NO:42) | FDRGNSFPY (SEQ ID NO:20) | WGHGT LVTVSA (SEQ ID NO:43) |
| 5-G17 (heavy chain variable region: SEQ ID NO:38) | EVKLVESGG GLVKPGGSL KLSCAAS(SE Q ID NO:44) | GFTISDY (SEQ ID NO: 21) | GMAWVRQ APGKGPEW VAFI(SEQ ID NO:45) | SNLAYS(S EQ ID NO: 22) | IHYVDTVTGRFTI SRENAKNTLYLE MRSLRSEDTAM YYCAR(SEQ ID NO:46) | MYYGNALDY ( SEQ ID NO: 23) | WGQGT SVTVSS (SEQ ID NO:47) |

| Light chain CDR sequences (Chothia numbering) | | | | | | | |
|---|---|---|---|---|---|---|---|
| Antibody name | FR1 | CDR1 | FR2 | CDR2 | FR3 | CDR3 | FR4 |
| 5-I10 (light chain variable region: SEQ ID NO:37) | DIVMTQSPSS LTVTVGEKV TMTC(SEQ ID NO:48) | KSSQSLL NSGNQK NYLT(SE Q ID NO:24) | WYQQKPGQ SPKLLIY(SE Q ID NO:49) | WASTRES ( SEQ ID NO:25) | GVPERFTGRGSG TDFTLTISSVQAE DLAVYYC(SEQ ID NO:50) | QNGYSYP FT(SEQ ID NO:26) | FGSGTNLEIK (SEQ ID NO:51) |
| 5-G17 (light chain variable region: SEQ ID NO:39) | DIVMTQSPSS LTVTTGEKVT MSC(SEQ ID NO:52) | KSSQNL LNSGNQ KNYLT(S EQ ID NO:27) | WYQQKPGQ PPKLLIY(SE Q ID NO:53) | WASTRES ( SEQ ID NO:28) | GVPDRFTGSGSG TDFTLTISSVQAE DLAVYYC(SEQ ID NO:54) | QNDYSFP LT(SEQ ID NO:29) | FGAGTKLEL K(SEQ ID NO:55) |

**Example 6. Expression of recombinant mouse monoclonal antibody**

The heavy and light chain V regions of 5-110 and 5-G17 were fused with mouse IgG2a-type heavy chain constant regions (Table 7, Mouse IGHC2A, SEQ ID NO:30) and mouse kappa-type light chain constant regions (Table 7, Mouse IGKC, SEQ ID NO:31), respectively, and a signal peptide was added to the N-terminal of the heavy and light chain sequences, respectively. The mouse heavy chain full-length genes (SEQ ID NOs: 32, 33) and light chain full-length genes (SEQ ID NOs: 34, 35) of 5-I10 and 5-G17 were synthesized and cloned into expression vectors, and verified by sequencing (Genewiz Biotechnology Co., Ltd., Suzhou). The constructed expression plasmid of each candidate molecule was amplified and extracted, verified by agarose gel electrophoresis, and used as a transfection material.

HEK293 cells were inoculated into shake flasks for suspension seed culture using serum-free and chemically defined media. One day before transfection, the amplified HEK293 cells were inoculated into new shake flasks and replaced with fresh medium. The heavy chain and light chain expression plasmids of 5-I10 and 5-G17 were co-transfected into HEK293 cells, cultured in shake flasks until the cell viability decreases, and the supernatant was collected for product purification. First, most of the cell debris and insoluble particles were removed by centrifugation and filtration of the supernatant, and then the feed liquid was loaded onto the Protein A column, where the antibody molecules bind to Protein A on the filler. Most impurities were removed after an equilibration step, the antibody proteins were eluted using a low pH eluent to and the fractions were collected. pH was adjusted and exchanged to a formulation buffer by ultrafiltration. Finally, the OD280 was determined to calculate the protein concentration. As shown in Figure 11, the electropherograms of monoclonal antibodies 5-I10 and 5-G17 on reducing SDS-PAGE showed that the positions of the heavy and light chains were consistent with the positions of the positive control IgG heavy and light chains, and the molecular weights were as expected. There was no obvious impurity band, and the protein purity was >95%.

Table 7

| Mouse IGKC | RADAAPTVSIFPPSSEQLTSGGASVVCFLNNFYPKDINVKWKIDGSERQNGV LNSWTDQDSKDSTYSMSSTLTLTKDEYERHNSYTCEATHKTSTSPIVKSFNR NEC(SEQ ID NO:30) |
|---|---|
| Mouse IGHC2A | AKTTAPSVYPLAPVCGDTTGSSVTLGCLVKGYFPEPVTLTWNSGSLSSGVHT FPAVLQSDLYTLSSSVTVTSSTWPSQSITCNVAHPASSTKVDKKIEPRGPTIKP CPPCKCPAPNLLGGPSVFIFPPKIKDVLMISLSPMVTCVVVDVSEDDPDVQIS WFVNNVEVLTAQTQTHREDYNSTLRVVSALPIQHQDWMSGKEFKCKVNN KALPAPIERTISKPKGSVRAPQVYVLPPPEEEMTKKQVTLTCMVTDFMPEDI YVEWTNNGKTELNYKNTEPVLDSDGSYFMYSKLRVEKKNWVERNSYSCS VVHEGLHNHHTTKSFSRTPG(SEQ ID NO:31) |

**Example 7. HEK293-hCLDN18.2 binding activity of the recombinant mouse monoclonal antibody**

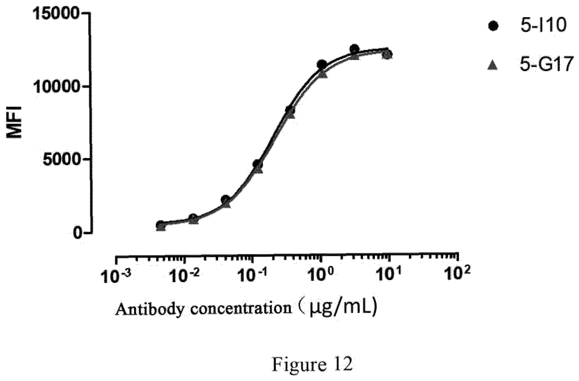

The binding of purified monoclonal antibodies 5-110 and 5-G17 to HEK293-hCLDN18.2 was verified by flow cytometry. HEK293-hCLDN18.2 was plated in a 96-well U-shaped plate ($1.5 \times 10^5$ cells/well), and the plate was washed once with PBS buffer containing 1% FBS. Purified monoclonal antibodies 5-I10 and 5-G17 were diluted with PBS (1% FBS) respectively, and a 3-fold gradient dilution was performed starting from the concentration of 10 μg/mL to obtain a total of 8 concentration points, they were then added to a 96-well U-plate at 100 μL/well and incubated at 4°C for 1 hour. Washing once with PBS (1% FBS), and 100 μL/well of Alexa488-labeled goat anti-mouse secondary antibody (eBioscience, A11001) 1:1000 diluted was added and incubated at 4°C for 1 hour. After washing twice with PBS (1% FBS), the cells were resuspended in 200 μL PBS (1% FBS) and detected by FACS. Figure 12 showed the binding curves of purified monoclonal antibodies 5-110 and 5-G17 to HEK293-hCLDN18.2, both antibodies can specifically bind to HEK293-hCLDN18.2 in a dose-dependent manner, with comparable binding abilities.

**Example 8. Positive staining of the recombinant mouse monoclonal antibody on gastric cancer FFPE tissue sections**

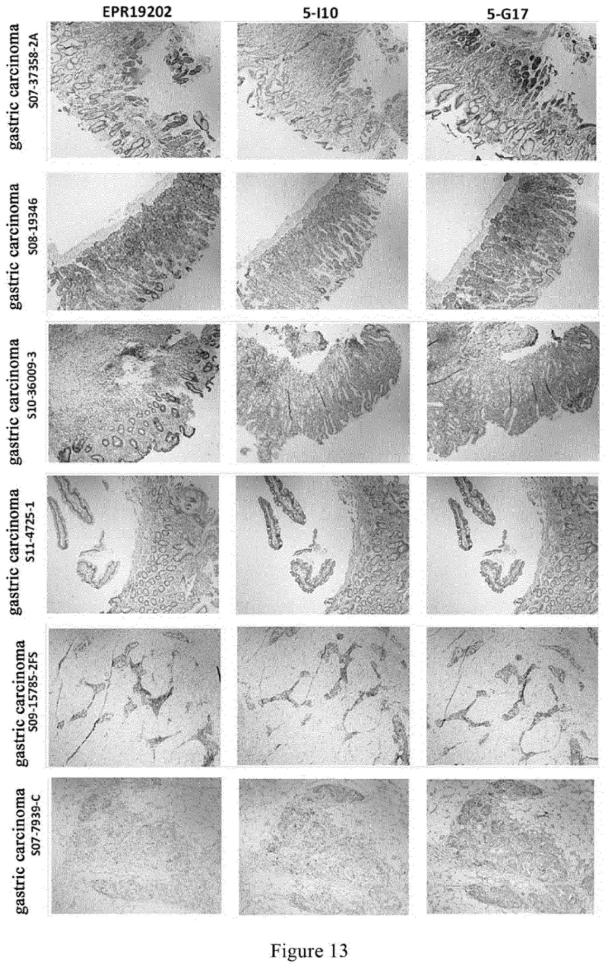

The CLDN18.2 IHC positive staining of purified monoclonal antibodies 5-110 and 5-G17 was verified on 6 cases of gastric cancer FFPE tissue sections, and EPR19202 was used as a positive control. The IHC staining procedure was the same as described in Example 4, wherein the working concentration of the positive control antibody EPR19202 in the primary antibody incubation step was 1:100 dilution, and the working concentration of purified antibodies 5-I10 and 5-G17 was 5 μg/mL. The staining results were shown in Figure 13. The recombinant and purified mouse IgG2a type 5-110 and 5-G17 monoclonal antibodies can be positively stained on several cases of gastric cancer FFPE tissue sections. The positive staining rates of 5-G17 and 5-110 reached 83.3% (5/6) and 66.7% (4/6), respectively, which were significantly higher than 50% (3/6), which was that of the positive control antibody EPR19202. On tissues S07-37358-2A, S08-19346 and S10-36009-3, gastric cancer cells form a relatively obvious gastric gland epithelial structure, and 5-110, 5-G17 and the positive control antibody EPR19202 all showed strong glandular epithelium-specific staining, but no staining on stromal cells. Gastric cancer cells form a gland-like structure on the S11-4725-1 tissue, but it was significantly different from the glandular structure on the aforementioned sections; the positive control SPR19202 had no obvious IHC staining, while 5-110 and 5-G17 had strong positive staining, which was in diffuse type, and both glandular epithelium and stromal cells were stained. Tumor cells on S09-15785-2FS and S07-7939-C sections had no obvious glandular structure formed, and positive control SPR19202, 5-110 and 5-G17 had no positive IHC staining on S09-15785-2FS. Only 5-G17 showed weak IHC positive staining on S07-7939-C.

**Sequence Listing**

SEQ ID NO:1

Atggccgtgactgcctgtcagggcttggggttcgtggtttcactgattgggattgcgggcatcattgctgccacctgcatggaccagtggagcacccaagacttgtacaa

caaccccgtaacagctgttttcaactaccaggggctgtggcgctcctgtgtccgagagagctctggcttcaccgagtgccggggctacttcaccctgctggggctgcca
gccatgctgcaggcagtgcgagccctgatgatcgtaggcatcgtcctgggtgccattggcctcctggtatccatctttgccctgaaatgcatccgcattggcagcatgga
ggactctgccaaagccaacatgacactgacctccgggatcatgttcattgtctcaggtctttgtgcaattgctggagtgtctgtgtttgccaacatgctggtgactaacttctg
gatgtccacagctaacatgtacaccggcatgggtgggatggtgcagactgttcagaccaggtacacatttggtgcggctctgttcgtgggctgggtcgctggaggcctc
acactaattgggggtgtgatgatgtgcatcgcctgccggggcctggcaccagaagaaaccaactacaaagccgtttcttatcatgcctcaggccacagtgttgcctacaa
gcctggaggcttcaaggccagcactggctttgggtccaacaccaaaaacaagaagatatacgatggaggtgcccgcacagaggacgaggtacaatcttatccttccaa
gcacgactatgtgtaa

SEQ ID NO:2
TQDLYNNPVTAVFNYQGLWRSCVRESSGFTECRGYFTLLGLPA

SEQ ID NO:3

MDIDPYKEFGASVELLSFLPSDFFPSIRDLLDTASALYREALESPEHCSPHHTALRQAVLCWGELMNLATWV
GSNLEDGGGGSGGGGTQDLYNNPVTAVFNYQGLWRSCVRESSGFTECRGYFTLLGLPAGGGGSGGGGSRE
LVVSYVNIMGLKIRQLLWFHISCLTFGRETVLEYLVSFGVWIRTPPAYRPPNAPILSTLPETTVVRGGSHHH
HHH

SEQ ID NO:4

Atggccgtgactgcctgtcagggcttggggttcgtggtttcactgattgggattgcgggcatcattgctgccacctgcatggaccagtggagcacccaagacttgtacaa
caaccccgtaacagctgttttcaactaccaggggctgtggcgctcctgtgtccgagagagctctggcttcaccgagtgccggggctacttcaccctgctggggctgcca
gccatgctgcaggcagtgcgagccctgatgatcgtaggcatcgtcctgggtgccattggcctcctggtatccatctttgccctgaaatgcatccgcattggcagcatgga
ggactctgccaaagccaacatgacactgacctccgggatcatgttcattgtctcaggtctttgtgcaattgctggagtgtctgtgtttgccaacatgctggtgactaacttctg
gatgtccacagctaacatgtacaccggcatgggtgggatggtgcagactgttcagaccaggtacacatttggtgcggctctgttcgtgggctgggtcgctggaggcctc
acactaattgggggtgtgatgatgtgcatcgcctgccggggcctggcaccagaagaaaccaactacaaagccgtttcttatcatgcctcgggccacagtgttgcctaca
agcctggaggcttcaaggccagcactggctttgggtccaacaccaaaaacaagaagatatacgatggaggtgcccgcacagaggacgaggtacaatcttatccttcca
agcacgactatgtgtaa

SEQ ID NO:5

atgtccaccaccacatgccaagtggtggcgttcctcctgtccatcctggggctggccggctgcatcgcggccaccgggatggacatgtggagcacccaggacctgta

cgacaaccccgtcacctccgtgttccagtacgaagggctctggaggagctgcgtgaggcagagttcaggcttcaccgaatgcaggcccctatttcaccatcctgggactt

ccagccatgctgcaggcagtgcgagccctgatgatcgtaggcatcgtcctgggtgccattggcctcctggtatccatctttgccctgaaatgcatccgcattggcagcat

ggaggactctgccaaagccaacatgacactgacctccgggatcatgttcattgtctcaggtctttgtgcaattgctggagtgtctgtgtttgccaacatgctggtgactaact

tctggatgtccacagctaacatgtacaccggcatgggtggggatggtgcagactgttcagaccaggtacacatttggtgcggctctgttcgtgggctgggtcgctggagg

cctcacactaattggggtgtgatgatgtgcatcgcctgccggggcctggcaccagaagaaaccaactacaaagccgtttcttatcatgcctcaggccacagtgttgcct

acaagcctggaggcttcaaggccagcactggctttgggtccaacaccaaaaacaagaagatatacgatggaggtgcccgcacagaggacgaggtacaatcttatcctt

ccaagcacgactatgtgtaa

SEQ ID NO:6

atgtcggtgaccgcctgccagggcttggggtttgtggtgtcactgatcgggtttgcgggcatcattgcagccacttgtatggaccagtggagcacccaggatttatacaa

caacccggtgaccgctgtattcaactaccaagggctatggcgttcatgcgtccgagagagctctggcttcaccgagtgccgaggctacttcaccctgttggggttgcca

gccatgctgcaagctgtacgagccctgatgatcgtgggcattgttctgggggtcatcggtatcctcgtgtccatcttcgccctgaagtgcattcgcattggtagcatggatg

actctgccaaggccaagatgactctgacttctgggatcttgttcatcatctccggcatctgtgcaatcattggtgtgtctgtgtttgccaacatgctggtgaccaacttctggat

gtccacagctaacatgtacagcggcatgggcggcatgggtggcatggtgcagaccgttcagaccaggtacacctttggtgcagctctgttcgtgggctgggttgctgga

ggcctcaccctgattggggggagtgatgatgtgcatcgcctgccgtggcctgacaccagatgacagcaacttcaaagctgtgtcttaccatgcctctggccaaaatgttgc

ctacaggcctggaggctttaaggccagcactggctttgggtccaacaccagaaacaagaagatctacgatgggggtgcccgcacagaagacgatgaacagtctcatc

ctaccaagtatgactatgtgtag

SEQ ID NO:7

atggccaccaccacgtgccaggtggtagggcttctcctgtccctcctgggtctggccggctgcatagccgccactgggatggacatgtggagcactcaagacctgtat

gacaacccagtcaccgccgtgttccagtatgaagggctctggaggagttgcgtgcaacagagctcggggttcaccgagtgccggccatacttcaccatcctgggcc'tt

ccagccatgctgcaagctgtacgagccctgatgatcgtgggcattgttctgggggtcatcggtatcctcgtgtccatcttcgccctgaagtgcattcgcattggtagcatgg

atgactctgccaaggccaagatgactctgacttctgggatcttgttcatcatctccggcatctgtgcaatcattggtgtgtctgtgtttgccaacatgctggtgaccaacttctg

gatgtccacagctaacatgtacagcggcatgggcggcatgggtggcatggtgcagaccgttcagaccaggtacacctttggtgcagctctgttcgtgggctgggttgct

ggaggcctcaccctgattggggagtgatgatgtgcatcgcctgccgtggcctgacaccagatgacagcaacttcaaagctgtgtcttaccatgcctctggccaaaatgt

tgcctacaggcctggaggctttaaggccagcactggctttgggtccaacaccagaaacaagaagatctacgatgggggtgcccgcacagaagacgatgaacagtctc

atcctaccaagtatgactatgtgtag

SEQ ID NO:8

atggaatggacctgggtctttctcttcctcctgtcagtaactgcaggtgtccactcccaggttcagctgcagcagtctggagctgagctgatgaagcctggggcctcagtg

aagatatcctgcaaggctactggctacacattcagtagctactggatagagtgggtaaagcagaggcctggacatggccttgagtggattggagagattttacctggaag

tggtagtactaactacaatgagaagttcaagggcaaggccacattcactgcagatacatcctccaacacagcctacatgcaactcagcagcctgacatctgaggactctg

ccgtctattactgtgcaagatatgattacccctggtttgcttactggggccaagggactctggtcactgtctctgcagcctctacaaagggccccagcgtgtttccactggct

ccctcctaagagcacaagcggcggcaccgctgccctgggctgtctggtgaaggactactttccagagcctgtgacagtgagctggaattccggcgctctgacctctg

gcgtgcacacctttccagccgtgctgcagtcttccggcctgtactccctgtctagcgtggtgaccgtgcccagctcctctctgggcacccagacatatatctgcaacgtga

atcacaagccttccaatacaaaggtggacaagaaggtggagccaaagtcctgtgacaagacccatacatgccccccatgtcctgctcccgagctgctgggcggccctt

ccgtgttcctgtttcccccaaagcccaaggatacctgatgatcagcagaaccccagaggtgacatgcgtggtggtggacgtgtcccatgaggatcccgaggtgaagtt

caactggtacgtggacggcgtggaggtgcataacgccaagacaaagcctagagaggagcagtacaactccacctaccgggtggtgtccgtgctgaccgtgctgcac

caggactggctgaatggcaaggagtacaagtgcaaggtgtctaataaggccctgcctgctccaatcgagaagacaatctctaaggctaagggccagcctctcgggagcc

ccaggtgtataccctgcctccatccagagacgagctgaccaagaatcaggtgtctctgacatgcctggtgaagggcttctatccatccgatatcgctgtggagtgggaga

gcaatggccagcctgagaacaattataagacaaccccacctgtgctggattctgacggcagcttttcctgtattccaagctgaccgtggataagtctagatggcagcag

ggcaacgtgttctcctgtagcgtgatgcacgaggcactgcataatcactacacccagaagtcactgtcactgagtccaggcaaataa

SEQ ID NO:9

atgcattttcaagtgcagattttcagcttcctgctaatcagtgcctcagtcataatgtccagaggacaaattgttctcacccagtctccagcaatcatgtctgcatctccaggg
gagaaggtcaccataacctgcagtgccagctcaagtgtaagttacatgcactggttccagcagaagccaggcacttctcccaaactctggatttatagcacatccaacct
ggcttctggagtccctgctcgcttcagtggcagtggatctgggacctcttactctctcacaatcagccgaatggaggctgaagatgctgccacttattactgccagcaaag
gagtagttacccacccacgttcggagggggggaccaagctggaaataaagagaaccgtggctgccccaagcgtgtttatcttccctccatctgatgagcagctgaagtct
ggcacagctagcgtggtgtgcctgctgaataacttctaccccagagaggccaaggtgcagtggaaggtggataacgctctgcagtctggcaactcccaggagtctgtg
acagagcaggattccaaggacagcacatactccctgtctagcaccctgacactgagcaaggctgactacgagaagcacaaggtgtacgcttgcgaggtcactcatca
gggactgtcatctcctgtcactaagagttttaatcgcggcgagtgttga

SEQ ID NO:10

atgggatggagctgtatcatcctcttcttggtagcaacagctacaggtgtccactcccaggtccaactgcagcagcctggggctgagctggtgaggcctggggcttcag
tgaagctgtcctgcaaggcttctggctacaccttcaccagctactggataaactgggtgaagcagaggcctggacaaggccttgagtggatcggaaatatttatccttctg
atagttatactaactacaatcaaaagttcaaggacaaggccacattgactgtagacaaatcctccagcacagcctacatgcagctcagcagcccgacatctgaggactct
gcggtctattactgtacaagatcgtggagggggtaactcctttgactactggggccaaggcaccactctcacagtctcctcagcctctacaaagggccccagcgtgtttcc
actggctccctcctctaagagcacaagcggcggcaccgctgccctgggctgtctggtgaaggactactttccagagcctgtgacagtgagctggaattccggcgctctg
acctctggcgtgcacaccttccagccgtgctgcagtcttccggcctgtactccctgtctagcgtggtgaccgtgcccagctcctctctgggcacccagacatatatctgc

aacgtgaatcacaagccttccaatacaaaggtggacaagaaggtggagccaaagtcctgtgacaagacccatacatgcccccatgtcctgctcccgagctgctgggc
ggcccttccgtgttcctgtttccccaaagcccaaggatacctgatgatcagcagaaccccagaggtgacatgcgtggtggtggacgtgtcccatgaggatcccgag
gtgaagttcaactggtacgtggacggcgtggaggtgcataacgccaagacaaagcctagagaggagcagtacaactccacctaccgggtggtgtccgtgctgaccgt
gctgcaccaggactggctgaatggcaaggagtacaagtgcaaggtgtctaataaggccctgcctgctccaatcgagaagacaatctctaaggctaagggccagcctc
gggagccccaggtgtataccctgcctccatccagagacgagctgaccaagaatcaggtgtctctgacatgcctggtgaagggcttctatccatccgatatcgctgtgga
gtgggagagcaatggccagcctgagaacaattataagacaaccccacctgtgctggattctgacggcagcttttcctgtattccaagctgaccgtggataagtctagatg
gcagcagggcaacgtgttctcctgtagcgtgatgcacgaggcactgcataatcactacacccagaagtcactgtcactgagtccaggcaaataa

SEQ ID NO:11

atggaatcacagactcaggtcctcatgtccctgctgttctgggtatctggtacctgtggggacattgtgatgacacagtctccatcctccctgactgtgacagcaggagag
aaggtcactatgagctgcaagtccagtcagagtctgttaaacagtggaaatcaaaagaactacttgacctggtaccagcagaaaccagggcagcctcctaaactgttgat
ctactgggcatccactagggaatctggggtccctgatcgcttcacaggcagtggatctgggacagatttcactctcaccatcagcagtgtgcaggctgaagacctggca
gtttattactgtcagaatgattatagttatccattcacgttcggctcggggacaaagttggaaataaagagaaccgtggctgccccaagcgtgtttatcttccctccatctgat
gagcagctgaagtctggcacagctagcgtggtgtgcctgctgaataacttctaccccagagaggccaaggtgcagtggaaggtggataacgctctgcagtctggcaac
tcccaggagtctgtgacagagcaggattccaaggacagcacatactccctgtctagcaccctgacactgagcaaggctgactacgagaagcacaaggtgtacgcttgc
gaggtcactcatcagggactgtcatctcctgtcactaagagttttaatcgcggcgagtgttga

SEQ ID NO:12

atggattggctgtggaacttgctattcctgatggcagctgcccaaagtatccaagcacagatccagttggtgcagtctggacctgagctgaagaagcctggagagacag
tcaagatctcctgcaaggcttctgggtataccttcacaaactatggaatgaactgggtgaagcaggctccaggaaagggtttaaagtggatgggctggataaacaccaa
cactggagagccaacatatgctgaagagttcaagggacgggtttgccttctctttggaaacctctgccagcactgcctatttgcagatcaacaacctcaaaaatgaggaca
cggctacatatttctgtgtgcaagactgggtttggtaatgctatggactactggggtcaaggaacctcagtcaccgtctcctcagcctctacaaagggcccccagcgtgtttcc
actggctccctcctctaagagcacaagcggcggcaccgctgccctgggctgtctggtgaaggactactttccagagcctgtgacagtgagctggaattccggcgctctg
acctctggcgtgcacacctttccagccgtgctgcagtcttccggcctgtactccctgtctagcgtggtgaccgtgcccagctcctctctgggcacccagacatatatctgc
aacgtgaatcacaagccttccaatacaaaggtggacaagaaggtggagccaaagtcctgtgacaagacccatacatgccccccatgtcctgctcccgagctgctgggc
ggcccttccgtgttcctgtttccccccaaagcccaaggataccctgatgatcagcagaacccccagaggtgacatgcgtggtggtggacgtgtcccatgaggatcccgag
gtgaagttcaactggtacgtggacggcgtggaggtgcataacgccaagacaaagcctagagaggagcagtacaactccacctaccgggtggtgtccgtgctgaccgt
gctgcaccaggactggctgaatggcaaggagtacaagtgcaaggtgtctaataaggccctgcctgctccaatcgagaagacaatctctaaggctaagggccagcctc
gggagccccaggtgtatacccctgcctccatccagagacgagctgaccaagaatcaggtgtctctgacatgcctggtgaagggcttctatccatccgatatcgctgtgga
gtgggagagcaatggccagcctgagaacaattataagacaaccccacctgtgctggattctgacggcagcttttttcctgtattccaagctgaccgtggataagtctagatg
gcagcagggcaacgtgttctcctgtagcgtgatgcacgaggcactgcataatcactacacccagaagtcactgtcactgagtccaggcaaataa

SEQ ID NO:13

atggaatcacagactcaggtcctcatgtccctgctgttctgggtatctggtacctgtggggacattgtgatgacacagtctccatcctccctgactgtgacagcaggagag
aaggtcactatgagctgcaagtccagtcagagtctgttaaacagtggaaatcaaaagaactacttgacctggtaccagcagaaaccagggcagcctcctaaactgttgat
ctactgggcatccactagggaatctggggtccctgatcgcttcacaggcagtggatctgggaacagatttcactctcaccatcagcagtgtgcaggctgaagacctggca
gtttattactgtcagaatgattatagttatccgctcacgttcggtgctgggaccaagctggagctgaagagaaccgtggctgcccccaagcgtgtttatcttccctccatctga
tgagcagctgaagtctggcacagctagcgtggtgtgcctgctgaataacttctaccccagagaggccaaggtgcagtggaaggtggataacgctctgcagtctggcaa
ctcccaggagtctgtgacagagcaggattccaaggacagcacatactccctgtctagcaccctgacactgagcaaggctgactacgagaagcacaaggtgtacgcttg
cgaggtcactcatcagggactgtcatctcctgtcactaagagttttaatcgcggcgagtgttga

SEQ ID NO:14

GAGATCCAGCTGCAGCAGTCTGGAGCTGAGCTGGTGAAGCCTGGGGCTTCAGTGAAGATATCCTGCAA
GGCTCCTGGTTACTCATTCACTGACTACAATATGAACTGGGTGAAGCAGAGCCATGGAAAGAGCCTTG

AGTGGATTGGAAATATTAATTCTTACTATGGTAGTACTAGCTACAATCAGAAGTTCAAGGGCAAGGCCA
CATTGACTGTAGACAGATCTTCCAGCACAGCCTACATGCAGCTCAACAGCCTGACATCTGAGGACTCTG
CAGTCTATTACTGTACAAACTTCGACAGGGGAAATTCGTTCCCTTACTGGGGCCACGGGACTCTGGTCA
CTGTCTCTGCA

SEQ ID NO:15

GAGGTGAAACTGGTGGAGTCTGGGGGAGGCTTAGTGAAGCCTGGAGGGTCCCTGAAACTCTCCTGTG
CAGCCTCTGGATTCACTATCAGTGACTACGGAATGGCGTGGGTTCGACAGGCTCCAGGGAAGGGGCCT
GAGTGGGTAGCATTCATTAGTAATTTGGCATATAGTATCCACTATGTAGACACTGTGACGGGCCGATTCA
CCATCTCTAGAGAGAATGCCAAGAACACCCTGTACCTGGAAATGCGTAGTCTGAGGTCTGAGGACACA
GCCATGTATTACTGTGCAAGAATGTACTATGGGAATGCTTTGGACTACTGGGGTCAAGGAACCTCAGTC
ACCGTCTCCTCA

SEQ ID NO:16

GACATTGTGATGACACAGTCTCCATCCTCCCTGACTGTGACAGTAGGAGAGAAGGTCACTATGACCTG
CAAGTCCAGTCAGAGTCTGTTAAACAGTGGAAATCAAAAGAACTATTTGACCTGGTACCAGCAGAAAC
CAGGGCAGTCTCCTAAACTGTTGATCTACTGGGCATCCACTAGGGAATCTGGGGTCCCTGAGCGCTTCA
CAGGCAGAGGATCTGGAACAGATTTCACTCTCACCATCAGCAGTGTGCAGGCTGAAGACCTGGCAGTT
TATTACTGTCAGAATGGTTATAGTTATCCATTCACGTTCGGCTCGGGGACAAACTTGGAAATAAAA

SEQ ID NO:17

GACATTGTGATGACACAGTCTCCATCCTCCCTGACTGTGACAACAGGAGAGAAGGTCACTATGAGTTG
CAAGTCCAGTCAGAATCTGTTAAACAGTGGAAATCAAAAGAACTACTTGACCTGGTACCAGCAGAAAC
CAGGGCAGCCTCCTAAACTGTTGATCTACTGGGCATCCACTAGGGAATCTGGGGTCCCTGATCGCTTCA
CAGGCAGTGGATCTGGAACAGATTTCACTCTCACCATCAGCAGTGTGCAGGCTGAAGACCTGGCAGTT
TATTACTGTCAGAATGATTATAGTTTTCCTCTCACGTTCGGTGCTGGGACCAAGCTGGAGCTGAAA

SEQ ID NO:18
GYSFTDY

SEQ ID NO:19
NSYYGS

SEQ ID NO:20
FDRGNSFPY

SEQ ID NO:21
GFTISDY

SEQ ID NO:22
SNLAYS

SEQ ID NO:23
MYYGNALDY

SEQ ID NO:24
KSSQSLLNSGNQKNYLT

SEQ ID NO:25
WASTRES

SEQ ID NO:26
QNGYSYPFT

SEQ ID NO:27
KSSQNLLNSGNQKNYLT

SEQ ID NO:28
WASTRES

SEQ ID NO:29
QNDYSFP

SEQ ID NO:30

RADAAPTVSIFPPSSEQLTSGGASVVCFLNNFYPKDINVKWKIDGSERQNGVLNSWTDQDSKDSTYSMSST
LTLTKDEYERHNSYTCEATHKTSTSPIVKSFNRNEC

SEQ ID NO:31


AKTTAPSVYPLAPVCGDTTGSSVTLGCLVKGYFPEPVTLTWNSGSLSSGVHTFPAVLQSDLYTLSSSVTVTS
STWPSQSITCNVAHPASSTKVDKKIEPRGPTIKPCPPCKCPAPNLLGGPSVFIFPPKIKDVLMISLSPMVTCVV
VDVSEDDPDVQISWFVNNVEVLTAQTQTHREDYNSTLRVVSALPIQHQDWMSGKEFKCKVNNKALPAPIE
RTISKPKGSVRAPQVYVLPPPEEEMTKKQVTLTCMVTDFMPEDIYVEWTNNGKTELNYKNTEPVLDSDGS
YFMYSKLRVEKKNWVERNSYSCSVVHEGLHNHHTTKSFSRTPG

SEQ ID NO:32


atgggctggagctgcatcatcctctttctggtggctactgctaccggcgtgcactccgagattcaactccagcagagcggagccgagctggtgaaacccggcgcctcc
gtcaaatcagctgcaaagcccccggatacagcttcaccgactacaacatgaactgggtcaagcagtcccacggcaagtctctggagtggatcggcaacatcaacag
ctactacggatccacctcctacaaccagaagttcaagggcaaggccaccctcaccgtggataggtccagctccaccgcctacatgcagctgaactccctcaccagcga
agactccgctgtgtactactgcaccaacttcgacagaggcaacagctttccctattggggacatggaactctggtcaccgtgagcgccgccaaaaccaccgctcctagc
gtctaccccctcgctcccgtgtgtggcgacaccactggaagctccgtgactctgggctgtctcgtgaagggatacttccccgagcccgtgactctgacttggaattccgg
ctctctgagctccggcgtccacactttccccgccgtgctgcagtccgatctgtacactctgagcagcagcgtgaccgtcacctccagcacttggccttcccagtccatcac
ttgcaacgtggctcaccccgccagcagcaccaaggtcgacaagaagatcgagcctaggggacccactatcaaaccttgccctccttgcaaatgtcccgcccccaatct
gctgggcggacccagcgtgttcatcttcccccccaagatcaaggacgtgctcatgatctctctgagccccatggtgacttgcgtcgtggtcgacgtctccgaggatgatc
ccgacgtgcagattagctggttcgtgaacaacgtcgaggtcctgactgctcagacccaaacccataggggaggactacaactccactctgagggtcgtcagcgctctgc
ccattcagcaccaagactggatgtccggaaaggagttcaagtgcaaggtcaacaacaaggctctccccgcccccatcgagagaaccatcagcaaacccaagggcag
cgtgagagctcctcaagtgtatgtgctgcccccccccgaagaagagatgactaagaagcaagtgaccctcacttgcatggtcaccgatttcatgcccgaggacatctac
gtggagtggaccaacaacggcaagaccgagctcaattacaaaaacaccgagcccgtgctggacagcgacggctcctactttatgtactccaagctgagggtggagaa
gaagaactgggtcgagagaaactcctacagctgctccgtggtccatgagggactgcacaaccaccataccaccaagtccttctccagaactcccggctaa


SEQ ID NO:33


atgggctggagctgtatcatcctctttctggtcgccaccgctaccggagtccactccgaggtcaagctcgtggaaagcggaggcggactggtgaagcccggcggcag
cctcaagctgagctgcgccgccgcttccggcctttaccatcagcgactatggcatggccttgggtcagacaagcccccggcaaaggacccgagtgggtggccttcatcagcaa
cctcgcctattccatccactacgtcgataccgtcaccggaaggttcaccatttctagggagaacgccaagaacactctgtatctggagatgaggtctctgagaagcgagg
ataccgccatgtactattgcgctaggatgtattacggaaacgctctggactactgggggccaaggcacctccgtcactgtgtcctccgccaagaccactgctcctagcgtg
tatcctctggctcccgtgtgcggagacactaccggaagcagcgtcactctgggatgcctcgtcaaaggctacttccccgagcccgtcactctgacttggaactccggatc
tctgagcagcggagtccacacctttcccgccgtcctccaaagcgatctgtacactctgagctccagcgtgactgtcacttccagcacttggcccagccagagcatcactt
gcaacgtcgcccaccccgccagctccaccaaggtcgacaagaagatcgaacccagaggacctaccatcaagccttgccctccttgcaaatgccccgctcctaatctgc
tcggaggacccagcgtcttcattttccccccaagatcaaagacgtgctcatgatctctctgagccccatggtcacttgcgtcgtggtcgacgtctccgaagatgacccg
atgtccagatcagctggttcgtcaacaacgtggaagtcctgaccgcccagacccagacccataggggaagactacaactccactctgagagtcgtcagcgctctgccca
ttcagcaccaagactggatgtccggaaaggagttcaagtgcaaggtcaacaacaaggctctgcccgcccccatcgagaggaccattagcaagcctaagggctccgtg
agagcccctcaagtgtacgtcctccctcctccccgaggaagagatgaccaagaagcaagtgactctgacttgcatggtgaccgactttatgcccgaggatatctacgtcg
agtggaccaacaacggcaagaccgagctcaactacaagaataccgagcccgtgctggacagcgacggctcctactttatgtactccaagctgagggtcgagaagaaa
aactgggtggagagaaattcctacagctgctccgtcgtgcatgagggactgcacaaccaccataccactaagagcttctctaggactcccggataa


SEQ ID NO:34

atggagagccagacccaagtgctgatgtctctgctcttctgggtcagcggcacttgcggcgatatcgtcatgactcagtcccccagctctctgactgtcactgtcggcga
aaaggtgaccatgacttgcaagagcagccagtccctcctcaattccggcaaccagaagaactacctcacttggtaccagcagaagcccggacagagccctaagctgc
tgatctactgggcctccactagagagtccggagtgcccgagaggtttaccggcagaggctccggcaccgatttcaccctcaccatcagcagcgtgcaagccgaggat
ctggccgtctactactgccagaacggatactcctaccccttcaccttcggctccggcactaatctggagatcaaaagagccgacgccgctcccaccgtcagcatcttccc
tcctagctccgagcaactgacttccggcggagccagcgtggtgtgctttctgaacaacttctatcccaaagacattaacgtcaagtggaagatcgatggcagcgagagg
cagaatgccgtgctgaacagctggactgaccaagatagcaaagactccacctactccatgagctccaccctcactctcaccaaggatgagtacgagaggcacaactcc
tacacttgcgaagccacccacaagacctccaccagccccatcgtgaagagcttcaataggaacgagtgctaa

SEQ ID NO:35

atggagtcccagacccaagtgctgatgtctctgctgttttgggtcagcggaacttgcggcgatatcgtcatgactcagagccccagcagcctcaccgtgactactggcga
aaaggtcaccatgagctgcaaaagcagccagaacctcctcaacagcggcaaccagaagaactacctcacttggtaccagcagaagcccggacagccccctaagctc
ctcatctactgggccagcactagggagagcggagtgcccgataggttcaccggcagcggaagcggcaccgatttcactctgactatcagctccgtgcaagccgaaga
tctggccgtctactactgccagaacgactactccttccctctgaccttcggagccggaaccaagctcgagctgaagagagccgatgccgcccccactgtcagcatctttc
ctcccagctccgaacagctgactagcggaggcgcctccgtggtgtgcttcctcaacaacttctatcccaaagatatcaacgtcaagtggaagatcgacggcagcgaga
gacagaacggcgtgctgaacagctggaccgaccaagatagcaaggactccacttatagcatgagcagcactctgactctgactaaggacgagtatgagaggcacaa
ctcctacacttgcgaggccactcacaaaacctccacctcccccatcgtcaagtccttcaataggaacgagtgttaa

SEO ID NO:36

EIQLQQSGAELVKPGASVKISCKAPGYSFTDYNMNWVKQSHGKSLEWIGNINSYYGSTSYNQKFKGKATL
TVDRSSSTAYMQLNSLTSEDSAVYYCTNFDRGNSFPYWGHGTLVTVSA

SEQ ID NO:37

EVKLVESGGGLVKPGGSLKLSCAASGFTISDYGMAWVRQAPGKGPEWVAFISNLAYSIHYVDTVTGRFTIS
RENAKNTLYLEMRSLRSEDTAMYYCARMYYGNALDYWGQGTSVTVSS

SEQ ID NO:38

DIVMTQSPSSLTVTVGEKVTMTCKSSQSLLNSGNQKNYLTWYQQKPGQSPKLLIYWASTRESGVPERFTGR
GSGTDFTLTISSVQAEDLAVYYCQNGYSYPFTFGSGTNLEIK

SEQ ID NO:39

DIVMTQSPSSLTVTTGEKVTMSCKSSQNLLNSGNQKNYLTWYQQKPGQPPKLLIYWASTRES
GVPDRFTGSGSGTDFTLTISSVQAEDLAVYYC QNDYSFPLTFGAGTKLELK

SEQ ID NO:40
EIQLQQSGAELVKPGASVKISCKAP

SEQ ID NO:41
NMNWVKQSHGKSLEWIGNI

SEQ ID NO:42
TSYNQKFKGKATLTVDRSSSTAYMQLNSLTSEDSAVYYCTN

SEQ ID NO:43
WGHGTLVTVSA

SEQ ID NO:44
EVKLVESGGGLVKPGGSLKLSCAAS

SEQ ID NO:45
GMAWVRQAPGKGPEWVAFI

SEQ ID NO:46
IHYVDTVTGRFTISRENAKNTLYLEMRSLRSEDTAMYYCAR

SEQ ID NO:47
WGQGTSVTVSS

SEQ ID NO:48
DIVMTQSPSSLTVTVGEKVTMTC

SEQ ID NO:49
WYQQKPGQSPKLLIY

SEQ ID NO:50
GVPERFTGRGSGTDFTLTISSVQAEDLAVYYC

SEQ ID NO:51
FGSGTNLEIK

SEQ ID NO:52
DIVMTQSPSSLTVTTGEKVTMSC

SEQ ID NO:53
WYQQKPGQPPKLLIY

SEQ ID NO:54
GVPDRFTGSGSGTDFTLTISSVQAEDLAVYYC

SEQ ID NO:55
FGAGTKLELK

**Claims**

1. A CLDN18.2 antibody comprising heavy chain CDRs and light chain CDRs,
   wherein the heavy chain CDRs are:

   CDR1 comprising the sequence as shown in SEQ ID NO:18, CDR2 comprising the sequence as shown in SEQ ID NO:19, and CDR3 comprising the sequence as shown in SEQ ID NO:20; or
   CDR1 comprising the sequence as shown in SEQ ID NO:21, CDR2 comprising the sequence as shown in SEQ ID NO:22, and CDR3 comprising the sequence as shown in SEQ ID NO:23,
   wherein the light chain CDRs are:
   CDR1 comprising the sequence as shown in SEQ ID NO:24, CDR2 comprising the sequence as shown in SEQ ID NO:25, and CDR3 comprising the sequence as shown in SEQ ID NO:26; or CDR1 comprising the sequence as shown in SEQ ID NO:27, CDR2 comprising the sequence as shown in SEQ ID NO:28, and CDR3 comprising the sequence as shown in SEQ ID NO:29.

2. The antibody of claim 1, comprising a heavy chain variable region as shown in SEQ ID NO:36 or SEQ ID NO:37.

3. The antibody of claim 1, comprising a light chain variable region as shown in SEQ ID NO:38 or SEQ ID NO:39.

4. The antibody of any one of claims 1-3, comprising a heavy chain variable region as shown in SEQ ID NO:36 and a light chain variable region as shown in SEQ ID NO:38.

5. The antibody of any one of claims 1-3, comprising a heavy chain variable region as shown in SEQ ID NO:37 and a light chain variable region as shown in SEQ ID NO:39.

6. The antibody of any one of claims 1-5, which is a chimeric antibody, a humanized antibody, or a fully human antibody.

7. The antibody of any one of claims 1-6, which is a full-length antibody.

8. The antibody of claim 7, which is an IgG antibody.

9. The antibody of any one of claims 1-6, which is an antibody fragment that binds to CLDN18.2.

10. The antibody of claim 9, which is Fab, Fab'-SH, Fv, scFv or (Fab')$_2$.

11. The antibody of any one of claims 1-8, which is a multispecific antibody or a bispecific antibody.

12. The antibody of claim 11, wherein the multispecific or bispecific antibody comprises a binding domain that binds to a second biomolecule, wherein the second biomolecule is a cell surface antigen.

13. The antibody of claim 12, wherein the cell surface antigen is a tumor antigen.

14. The antibody of claim 13, wherein the tumor antigen is selected from the group consisting of: CD3, CD20, FcRH5, HER2, LYPD1, LY6G6D, PMEL17, LY6E, CD19, CD33, CD22, CD79A, CD79B, EDAR, GFRA1, MRP4, RET, Steap1 and TenB2.

15. An immunoconjugate, comprising a therapeutic agent, an agonist of stimulator of interferon genes (STING) receptor, a cytokine, a radionuclide or an enzyme linked to the antibody of any one of claims 1-10.

16. The immunoconjugate of claim 15, wherein the therapeutic agent is a chemotherapeutic drug.

17. The immunoconjugate of claim 15, wherein the therapeutic agent is a cytotoxic agent.

18. A fusion protein or polypeptide comprising the antibody of any one of claims 1-10 or an antigen-binding fragment thereof.

19. A pharmaceutical composition comprising the antibody of any one of claims 1-14, the immunoconjugate of any one of claims 15-17, or the fusion protein or polypeptide of claim 18.

20. An isolated nucleic acid comprising a polynucleotide sequence encoding the amino acid sequence of any one of SEQ ID NOs: 18-29.

21. The nucleic acid of claim 20, comprising a polynucleotide sequence encoding the amino acid sequence of any one of SEQ ID NOs: 36-39.

22. The nucleic acid of claim 21, comprising a polynucleotide sequence encoding the antibody of any one of claims 1-10 or an antigen-binding fragment thereof.

23. A vector comprising the polynucleotide sequence of any one of claims 20-22.

24. A host cell comprising the polynucleotide sequence of any one of claims 20-22 or the vector of claim 23.

25. A method for preparing the antibody of any one of claims 1-10, comprising culturing the host cell of claim 24 and isolating the antibody from the culture.

26. Use of a pharmaceutical composition comprising the antibody of any one of claims 1-14, the immunoconjugate of any one of claims 15-17, or the fusion protein or polypeptide of claim 18 in the preparation of a reagent for diagnosing cancer.

27. Use of a pharmaceutical composition comprising the antibody of any one of claims 1-14, the immunoconjugate of any one of claims 15-17, or the fusion protein or polypeptide of claim 18 in the preparation of a medicament for treating a cancer.

28. The use of claim 26 or 27, wherein the cancer is a gastric cancer, a pancreatic cancer or an esophageal cancer.

29. A cancer detection reagent comprising the antibody of any one of claims 1-10 or an antigen-binding fragment thereof.

30. The cancer detection reagent of claim 29, wherein the antibody or antigen-binding fragment thereof is chemically labeled.

31. The cancer detection reagent of claim 30, wherein the label is an enzymatic label, a fluorescent label, an isotopic label or a chemiluminescent label.

32. A cancer diagnosis kit comprising the cancer detection reagent of any one of claims 29-31.

33. The kit of claim 32, wherein the cancer is a gastric cancer, a pancreatic cancer or an esophageal cancer.

Figure 1

Figure 2

Figure 3

NR-SDS-PAGE     R-SDS-PAGE

Figure 4

Figure 5

Figure 6

Figure 7

Figure 8

Figure 9

Figure 10

Figure 11

Figure 12

Figure 13

**EP 4 105 237 A1**

## INTERNATIONAL SEARCH REPORT

| International application No. |
| --- |
| **PCT/CN2021/076481** |

**A. CLASSIFICATION OF SUBJECT MATTER**

C07K 16/30(2006.01)i; A61K 39/395(2006.01)i; A61K 39/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; A61K; A61P

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB, NCBI, STNext, 中国生物序列检索系统, CHINESE PATENT BIOLOGICAL SEQUENCE RETRIEVAL SYSTEM, 上海诗健生物科技有限公司, 周清, 许传营, 粘伟红, 何向宇, 张新敏, 郑欣桐, 何峰, 肖婧, 密蛋白18.2, 密连接蛋白18.2, tight junction protein, Claudin18.2, CLDN18.2, CLD18A2, 抗体, 轻链, 重链, CDR, 胃癌, 胰腺癌, 食管癌, antibody, light chain, heavy chain, gastric cancer, pancreatic cancer, esophagus cancer, SEQ ID NOs:18-29、36-39

**C. DOCUMENTS CONSIDERED TO BE RELEVANT**

| Category* | Citation of document, with indication, where appropriate, of the relevant passages | Relevant to claim No. |
| --- | --- | --- |
| X | CN 110606891 A (L&L BIOPHARMA, CO., LTD.) 24 December 2019 (2019-12-24) claims 1-20 | 20, 23, 24 |
| A | WO 2014127785 A1 (GANYMED PHARMACEUTICALS AG et al.) 28 August 2014 (2014-08-28) entire document | 1-33 |
| A | WO 2020018852 A2 (ASKGENE PHARMA INC. et al.) 23 January 2020 (2020-01-23) entire document | 1-33 |
| A | CN 104321345 A (GANYMED PHARMACEUTICALS AG et al.) 28 January 2015 (2015-01-28) entire document | 1-33 |
| A | SAHIN, U. et al. "Claudin-18 SpliceVariant 2 is a Pan-Cancer Target Suitable for Therapeutic Antibody Development" *Clinical Cancer Research*, Vol. 14, No. 23, 01 December 2008 (2008-12-01), pp. 7624-7634 | 1-33 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| **23 April 2021** | **12 May 2021** |

| Name and mailing address of the ISA/CN | Authorized officer |
| --- | --- |
| **China National Intellectual Property Administration (ISA/CN)** **No. 6, Xitucheng Road, Jimenqiao, Haidian District, Beijing 100088 China** | |
| Facsimile No. **(86-10)62019451** | Telephone No. |

Form PCT/ISA/210 (second sheet) (January 2015)

## INTERNATIONAL SEARCH REPORT

International application No.

**PCT/CN2021/076481**

**C.** **DOCUMENTS CONSIDERED TO BE RELEVANT**

| Category* | Citation of document, with indication, where appropriate, of the relevant passages | Relevant to claim No. |
|---|---|---|
| A | TÜRECI, Ö. et al. "Characterization of Zolbetuximab in Pancreatic Cancer Models" *Oncoimmunology*, Vol. 8, No. 1, 10 November 2018 (2018-11-10), document number: e1523096 , pp. 1-10 | 1-33 |
| A | 徐良额等 (XU, Liang'e et al.). "CLDN18.2 蛋白在恶性肿瘤治疗中的研究进展 (Advances of CLDN18.2 Protein in the Therapy of Malignant Tumors)" 中国肿瘤临床 *(Chinese Journal of Clinical Oncology)*, Vol. 46, No. 6, 30 March 2019 (2019-03-30), pp. 311-315 | 1-33 |
| A | 陈思远等 (CHEN, Siyuan et al.). "紧密连接蛋白claudins 应用于肿瘤治疗的进展 (Advances in the Application of Claudins to Tumor Therapy)" 生物工程学报 *(Chinese Journal of Biotechnology)*, Vol. 35, No. 6, 25 June 2019 (2019-06-25), pp. 931-941 | 1-33 |

Form PCT/ISA/210 (second sheet) (January 2015)

**INTERNATIONAL SEARCH REPORT**

International application No.

**PCT/CN2021/076481**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

**INTERNATIONAL SEARCH REPORT**
Information on patent family members

International application No.

**PCT/CN2021/076481**

| Patent document cited in search report | | | Publication date (day/month/year) | Patent family member(s) | | | Publication date (day/month/year) |
|---|---|---|---|---|---|---|---|
| CN | 110606891 | A | 24 December 2019 | None | | | |
| WO | 2014127785 | A1 | 28 August 2014 | KR | 20150120994 | A | 28 October 2015 |
| | | | | JP | 2020125329 | A | 20 August 2020 |
| | | | | MX | 2015010724 | A | 11 April 2016 |
| | | | | WO | 2014127906 | A1 | 28 August 2014 |
| | | | | AU | 2014221035 | A1 | 10 September 2015 |
| | | | | US | 10314890 | B2 | 11 June 2019 |
| | | | | SG | 11201506317P | A | 29 September 2015 |
| | | | | US | 2019298803 | A1 | 03 October 2019 |
| | | | | RU | 2699549 | C2 | 06 September 2019 |
| | | | | RU | 2019124332 | A | 04 October 2019 |
| | | | | IL | 239981 | D0 | 31 August 2015 |
| | | | | AR | 094849 | A1 | 02 September 2015 |
| | | | | AU | 2014221035 | B2 | 04 October 2018 |
| | | | | CA | 2899754 | A1 | 28 August 2014 |
| | | | | JP | 2016510721 | A | 11 April 2016 |
| | | | | HK | 1213925 | A1 | 15 July 2016 |
| | | | | UA | 118664 | C2 | 25 February 2019 |
| | | | | JP | 2018197260 | A | 13 December 2018 |
| | | | | SG | 10201610412Y | A | 27 February 2017 |
| | | | | JP | 6694926 | B2 | 20 May 2020 |
| | | | | US | 2015374789 | A1 | 31 December 2015 |
| | | | | JP | 6389478 | B2 | 12 September 2018 |
| | | | | RU | 2015139901 | A | 24 March 2017 |
| | | | | CN | 105073777 | A | 18 November 2015 |
| | | | | US | 2021023177 | A1 | 28 January 2021 |
| | | | | US | 9770487 | B2 | 26 September 2017 |
| | | | | US | 2018000900 | A1 | 04 January 2018 |
| | | | | IL | 239981 | A | 31 August 2015 |
| WO | 2020018852 | A2 | 23 January 2020 | US | 2020040101 | A1 | 06 February 2020 |
| CN | 104321345 | A | 28 January 2015 | PT | 2847225 | T | 16 January 2020 |
| | | | | DK | 2847225 | T3 | 20 January 2020 |
| | | | | WO | 2013167259 | A1 | 14 November 2013 |
| | | | | HU | E049171 | T2 | 28 September 2020 |
| | | | | EP | 2847225 | B1 | 27 November 2019 |
| | | | | US | 2018319891 | A1 | 08 November 2018 |
| | | | | SI | 2847225 | T1 | 31 March 2020 |
| | | | | NZ | 718280 | A | 27 April 2018 |
| | | | | AU | 2013258432 | B2 | 01 June 2017 |
| | | | | UA | 116445 | C2 | 26 March 2018 |
| | | | | US | 9512232 | B2 | 06 December 2016 |
| | | | | AU | 2019222874 | A1 | 19 September 2019 |
| | | | | BR | 112014026755 | B1 | 16 June 2020 |
| | | | | JP | 2018052934 | A | 05 April 2018 |
| | | | | AR | 090973 | A1 | 17 December 2014 |
| | | | | AU | 2017216513 | B2 | 30 May 2019 |
| | | | | IL | 235261 | D0 | 31 December 2014 |
| | | | | JP | 2015517476 | A | 22 June 2015 |
| | | | | ZA | 201407336 | B | 27 July 2016 |
| | | | | EP | 2847225 | A1 | 18 March 2015 |

Form PCT/ISA/210 (patent family annex) (January 2015)

International application No.

**PCT/CN2021/076481**

| Patent document cited in search report | Publication date (day/month/year) | Patent family member(s) | | Publication date (day/month/year) |
|---|---|---|---|---|
| | | US 10053512 | B2 | 21 August 2018 |
| | | WO 2013167153 | A1 | 14 November 2013 |
| | | EP 3666796 | A1 | 17 June 2020 |
| | | SG 11201406472W | A | 27 November 2014 |
| | | RU 2661772 | C2 | 19 July 2018 |
| | | NZ 700823 | A | 29 July 2016 |
| | | ES 2763965 | T3 | 01 June 2020 |
| | | HR P20192347 | T1 | 03 April 2020 |
| | | US 2016333109 | A1 | 17 November 2016 |
| | | AU 2017216513 | A1 | 31 August 2017 |
| | | RS 59868 | B1 | 31 March 2020 |
| | | LT 2847225 | T | 11 May 2020 |
| | | US 2015147763 | A1 | 28 May 2015 |
| | | MX 2014013542 | A | 08 May 2015 |
| | | JP 6232646 | B2 | 22 November 2017 |
| | | HK 1202557 | A1 | 02 October 2015 |
| | | SG 10201606048P | A | 29 September 2016 |
| | | PL 2847225 | T3 | 18 May 2020 |
| | | JP 2019172675 | A | 10 October 2019 |
| | | BR 112014026755 | A2 | 11 July 2017 |
| | | AU 2013258432 | A1 | 30 October 2014 |
| | | BR 112014026755 | B8 | 07 July 2020 |
| | | MX 369740 | B | 20 November 2019 |
| | | CN 104321345 | B | 30 March 2018 |
| | | KR 20150008095 | A | 21 January 2015 |
| | | CN 108047331 | A | 18 May 2018 |
| | | JP 6514294 | B2 | 15 May 2019 |
| | | CA 2869725 | A1 | 14 November 2013 |
| | | RU 2014149332 | A | 27 June 2016 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6075181 A
- US 6150584 A
- WO 9316185 A
- EP 404097 A
- WO 199301161 A
- US 6248516 B1
- US 4816567 A
- US 5821337 A
- US 7527791 B
- US 6982321 B
- US 7087409 B
- US 5770429 A
- US 7041870 B
- US 20070061900
- US 7189826 B
- US 5750373 A
- US 20050079574 A
- US 20050119455 A
- US 20050266000 A
- US 20070117126 A
- US 20070237764 A
- US 20070292936 A
- US 20090002360 A
- WO 9308829 A
- WO 200908025 A
- WO 2009089004 A1
- US 5648237 A
- US 5789199 A
- US 5840523 A
- US 5959177 A
- US 6040498 A
- US 6420548 B
- US 7125978 B
- US 6417429 B
- US 6267958 B
- US 6171586 B
- WO 2006044908 A

### Non-patent literature cited in the description

- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **LI et al.** *Proc. Natl. Acad. Sci. USA,* 2006, vol. 103, 3557-3562
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **HUDSON et al.** *Nat. Med.,* 2003, vol. 9, 129-134
- **BOERNER et al.** *J. Immunol.,* 1991, vol. 147, 86
- **NI.** *Xiandai Mianyixue,* 2006, vol. 26 (4), 265-268
- **HOOGENBOOM et al.** Methods in Molecular Biology. Human Press, 2001, vol. 178, 1-37
- **MARKS ; BRADBURY.** Methods in Molecular Biology. Human Press, 2003, vol. 248, 161-175
- **LEE et al.** *J. Immunol. Methods,* 2004, vol. 284 (1-2), 119-132
- **LI et al.** *Nat. Biotech.,* 2006, vol. 24, 210-215
- **PERCIVAL-ALWYN J. et al.** *mAbs,* 2015, vol. 7 (1), 129-137
- **THORSTEN K. et al.** *Cancer Res,* 2011, vol. 71 (2), 515-527
- **ZHANG G F et al.** *Human Gene Ther.,* 1999, vol. 10, 1735-1737
- **LIU F et al.** *Gene Ther.,* 1999, vol. 6, 1258-1266
- **SOTOSHI N et al.** *J Immunol Method,* 2003, vol. 280, 59-72
- **EDWARD AG.** Antibodies: A laboratory manual. 2012
- **SAHIN. U. et al.** *Human Cancer Biology,* 2008, vol. 14 (23), 7624-7634
- **ZHU G et al.** *Sci Rep.,* 2019, vol. 9, 8420-8431